# EUROPEAN PATENT SPECIFICATION

(11) **EP 3 979 931 B1**
(45) Date of publication and mention of the grant of the patent: **25.11.2026**
(21) Application number: 20740073.0
(22) Date of filing: 05.06.2020
(51) Int. Cl.: A61B 17/70, A61B 17/80

(54) **ANCHORING DEVICE FOR USE IN SPINAL DEFORMITY CORRECTION SURGERY**
VERANKERUNGSVORRICHTUNG ZUR VERWENDUNG BEI OPERATIONEN ZUR KORREKTUR VON WIRBELSÄULENDEFORMATIONEN
DISPOSITIF D'ANCRAGE DESTINÉ À ÊTRE UTILISÉ DANS UNE CHIRURGIE DE CORRECTION DE DÉFORMATION RACHIDIENNE

(30) Priority: 06.06.2019 GB 201908098
(43) Date of publication of application: 13.04.2022
(73) Proprietor: Dodds, Michael Kenneth, Dublin, K36 V296 (IE)
(72) Inventor: Dodds, Michael Kenneth, Dublin, K36 V296 (IE)
(74) Representative: Linn, Samuel Jonathan
(86) International application number: PCT/GB2020/051370
(87) International publication number: WO 2020/245603

(56) References cited:
- EP-A1- 2 777 569
- WO-A1-2016/166448
- WO-A1-2016/166482
- US-A1- 2012 029 567

## Description

### TECHNICAL FIELD

This invention relates to an anchoring device for use in spinal deformity correction surgery. More particularly, though not exclusively, the invention relates to an anchoring device for use with a pedicle screw, especially by attachment to a pedicle screw, to provide a system for enhanced anchoring of sub-laminar bands used in corrective spinal vertebral fixation surgery. The invention further relates to such anchoring device and pedicle screw combinations.

### BACKGROUND OF THE INVENTION AND PRIOR ART

Scoliosis is a common form of spinal deformity. Surgical correction of the deformity generally involves the implantation of fixation anchors onto plural vertebrae, which are then drawn onto a stabilising rod, resulting in partial correction of the deformity and the rigid internal fixation of plural segments of the spinal column permitting auto-fusion of a given segment. The primary purpose of such surgery is often to prevent worsening of the deformity. A secondary purpose is usually to achieve some correction of the deformity in order to improve the global balance of the spinal column.

A variety of fixation anchors to the bone of the vertebrae can be used, depending on the mechanical need. These include pedicle screws, pedicle hooks, supra-laminar hooks, transverse process hooks, sub-laminar wires and sub-laminar tapes or bands, as well as combinations of various of the foregoing. The use of pedicle screws in conjunction with sub-laminar bands or tapes are often a preferred anchoring system in many practical instances.

Pedicle screws provide useful and efficient fixation points on the spine to permit stabilisation of the vertebrae relative to one another and also to permit a degree of manipulation of the vertebrae when correcting the spinal deformity. The pedicle screw heads (many common types of which are termed "tulip heads", by reason of their physical resemblance to the shape of a tulip flower head) are typically configured to permit direct engagement, along with neighbouring pedicle screw heads affixed to adjacent vertebrae, with an elongate stabilising rod, which provides a rigid construct and permits fusion of the spine in the desired position. Screws can be drawn towards a stabilising rod by translative movement using a reduction device, which engages the screw head and draws the screw to the rod by pulling the screw directly onto the rod, before they are finally secured to each other by a locking screw or nut.

However, the use of pedicle screws comes with some disadvantages, especially in the context of being attached directly to a stabilising rod. One fundamental weakness of screws is that the bone-screw interface can sometimes fail in response to excessive axial pull-out forces. This phenomenon may be commonly encountered in scoliosis surgery in particular, and it may significantly weaken the rigid spinal construct created as a means of treating it. This can sometimes be a particularly significant issue at the concavity of the apex of the scoliotic curve, owing to both the abnormal anatomy of the vertebrae in this region of the spine and also the greater translative distance the spine has to travel in being drawn onto a stabilising rod in this region.

Sub-laminar bands or tapes are better able to resist axial pull-out forces than screws, although they may have a limited ability to transfer torsional forces to the spine and to derotate deformities. Nevertheless, sub-laminar bands or tapes are frequently used to good effect in combination with pedicle screws, and such combinations may even be used in combination with stabilising rods to fulfil additional stabilisation or derotation functions. However, sub-laminar bands or tapes need anchoring to pedicle screws by some means.

There are known in the art devices for attaching sub-laminar bands or tapes to pedicle screw heads. One such known device is known as the "JAZZ Cap" [trade mark] system, from the company Implanet. Examples of this type of device are disclosed in WO 2016/166482 A1 and WO 2016/166448 A1. This known device employs a collar-like cap placed over a screw head and from a side of which protrudes a hoop-like extension through and within which a sub-laminar band or tape passes and is clamped, leaving the screw head to be unitable with a stabilising rod in a conventional manner. However, such an anchoring device has limited usefulness in practice, owing to its configuration and reliance on the conventional rod-locking screw/nut to anchor the collar-like cap onto the screw head.

Other known devices for attaching a sub-laminar band or tape to a pedicle screw head, which work on a similar principle to that employed in the devices of WO 2016/166482 A1 and WO 2016/166448 A1 referred to above, but are each constructed and operate slightly differently therefrom, are disclosed in EP 2 777 569 A1 and US 2012/029567 A1.

Indeed, the design and function of such known band/tape attachment devices are generally only to provide additional stability once a conventional stabilising rod has been secured to the screw, and deformity correction has been attempted. Their design and function is not to achieve additional deformity correction at the apex of the spinal curve or to increase axial load strength of the apical screws during correction manoeuvres. Furthermore, the anatomy of a scoliotic spine poses special technical difficulties, particularly around the apex of the concavity of the curve, such as:
(i) the pedicles may often be small and mis-shapen, making secure fixation of a pedicle screw more difficult and resulting in reduced pull-out strength;
(ii) the concavity pedicles are crowded together, making sequential placement of screws, and their subsequent attachment to a rod, more difficult;
(iii) the apical pedicle screws are typically furthest from the rod, thus requiring greater translation of the screws towards the rod, which further risks pull-out of screws and loss of fixation;
(iv) reduction devices attached to these screws are often convergent upon one another, making their use technically more difficult and load-sharing in spinal translation sub-optimal.

In view of the above shortcomings in the known art, there is a clinical need for a spinal implant system which has the benefits of a screw in terms of fixation and derotation capability, while maximising pull-out strength to allow superior translation of apical vertebrae towards, and/or rotation of vertebrae relative to, a stabilisation rod during deformity correction manoeuvres.

It is therefore a primary object of the present invention to address the above shortcomings of the known art and, in a major aspect, to provide an anchoring system that facilitates and renders more efficient and effective the anchoring of one or more sub-laminar bands or tapes to, or with respect to, a pedicle screw.

### SUMMARY OF THE INVENTION

The present invention relates to a device as claimed hereafter. Preferred embodiments of the invention are set forth in the dependent claims.

Accordingly, in a first aspect the present invention provides an anchoring device for anchoring one or more sub-laminar bands or tapes to a pedicle screw, the device comprising:
a first portion configured for attachment to a head portion of the pedicle screw by interengagement with said head portion; and
a plurality of second portions for anchoring thereto one or more sub-laminar bands or tapes;
wherein the first portion includes first interengagement means for interengagement with the head portion of the pedicle screw to effect the said interengagement attachment of the first portion of the anchoring device thereto;
wherein the said plurality of second portions are arranged equi-angularly or symmetrically around the first portion of the device, each second portion being configured and arranged for anchoring thereto a said sub-laminar band or tape, and each said second portion including, or being configured as, an apertured or hollow hoop- or loop-like portion or extension located on or extending from diametrically opposite sides of the first portion of the device and extending in radially opposite directions therefrom,
and further wherein each of the second portions has a general plane in which it lies and the second portions are configured such that the respective general planes of the second portions are substantially co-planar with each other.

In a second aspect, the present invention provides an anchoring arrangement for anchoring one or more sub-laminar bands or tapes with respect to one or more spinal vertebrae in corrective spinal vertebral surgery, the arrangement comprising:
a pedicle screw for insertion into a selected vertebral pedicle; and
an anchoring device according to the first aspect of the invention.

In some embodiments of the above-defined anchoring arrangement, the head portion of the pedicle screw may comprise second interengagement means configured for interengagement with the first interengagement means provided on the first portion of the anchoring device, whereby the first and second interengagement means are each configured so that they together, once they are interengaged with each other, effect the said interengagement attachment of the first portion of the anchoring device to the head portion of the pedicle screw.

Thus, provided for use with the anchoring device of the first aspect of the present invention or any embodiment thereof is a pedicle screw for insertion into a pedicle of a selected spinal vertebra in corrective spinal vertebral surgery, the pedicle screw comprising a head portion which includes second interengagement means configured for interengagement with the first interengagement means provided on the first portion of the anchoring device itself.

Moreover, in accordance with a third aspect of the present invention, there is provided a kit or set of component parts for use in corrective spinal vertebral surgery, the kit or set comprising:
at least one pedicle screw for insertion into a selected vertebral pedicle of one or more spinal vertebrae; and
at least one anchoring device according to the first aspect of the invention or any embodiment thereof.

In some embodiments of the above-defined kit or set of component parts, the kit or set may further comprise one or more sub-laminar bands or tapes for anchoring with respect to said one or more spinal vertebrae in said corrective spinal vertebral surgery.

Use of the present invention thus provides a method (not claimed) of anchoring one or more sub-laminar bands or tapes with respect to one or more spinal vertebrae in corrective spinal vertebral surgery, the method comprising:
providing at least one pedicle screw for insertion into a selected vertebral pedicle of one or more spinal vertebrae, and at least one anchoring device according to the first aspect of the invention or any embodiment thereof;
inserting the pedicle screw into a pedicle of a selected vertebra;
attaching the first portion of the anchoring device to the head portion of the pedicle screw by interengagement of the first interengagement means provided on the first portion of the anchoring device with the head portion of the pedicle screw; and
anchoring the one or more sub-laminar bands or tapes to the second portions of the anchoring device.

In practical examples of the above-defined method, the final step of anchoring the one or more sub-laminar bands or tapes to the second portions of the anchoring device may be accompanied or followed by one or more additional steps comprising:
tightening (e.g. to any extent necessary) the anchored sub-laminar band(s) or tape(s) in a tightened condition once it/they has/have been passed or threaded around at least one vertebra, and
locking or securing the anchored sub-laminar band(s) or tape(s) in a tightened condition, especially by means of a suitable locking or securement device (examples of which are well-known in the art).

In embodiments of the various aspects of the invention, the anchoring device may include any of various features, including any of various optional or preferred features, as defined and discussed hereinbelow, independently of any pedicle screw or sub-laminar band or tape or other component of any anchoring arrangement or kit forming other aspects of the invention. Likewise, in embodiments of the various aspects of the invention, the pedicle screw may include any of various features, including any of various optional or preferred features, as defined and discussed hereinbelow, independently of any anchoring device or sub-laminar band or tape or other component of any anchoring arrangement or kit forming other aspects of the invention.

In some embodiments of the anchoring device of the invention, the first portion may be configured for attachment to the head portion of the pedicle screw by interengagement of the first interengagement means with at least a radially outer sidewall portion of the screw head portion.

In some such embodiments, the first portion may be configured such that, upon interengagement of the first interengagement means with the outer sidewall portion of the screw head portion, at least a lower part of the first portion of the device adjacent the first interengagement means abuts or lies adjacent or engages a lower sidewall portion of the screw head portion.

In some embodiments of the anchoring device of the invention, the first portion of the device may be configured such that it is attachable to the head portion of the pedicle screw, via the interengagement therewith of the first interengagement means, by means of insertion of a screw-threaded shaft of the pedicle screw, which shaft extends from a base of the head portion, through a hole or aperture in the first portion of the device in a direction away from the first portion of the device and relative movement towards one another of the first portion of the device and the base of the screw head portion.

In some embodiments of the anchoring device of the invention, the first portion may comprise an apertured or hollow base portion and one or more, especially at least one pair of mutually oppositely arranged, attachment portions extending generally perpendicularly therefrom. The base portion may be apertured or hollow, e.g. with a suitably sized circular or other shaped hole or aperture therein, to allow passage therethrough of the screw-threaded shaft of the pedicle screw during the attachment of the anchoring device thereto.

In some embodiment forms, the attachment portions may be configured as a pair of diametrically oppositely arranged protruding lugs, tabs, arms or other engagement elements extending perpendicularly from the base portion on opposite, especially diametrically opposite, sides thereof, and may be disposed so as to be generally substantially parallel to one other and configured to interengage with the outer sidewall of the head portion of the screw on opposite, especially diametrically opposite, sides of the screw head portion.

In some such embodiment forms, the attachment portions may each terminate at a distal end thereof (that being the end thereof opposite a proximal end thereof at which the respective attachment portion is joined to the base portion of the first portion of the device) in a radially inwardly projecting interengagement nose portion, whereby the interengagement nose portions of the pair of attachment portions point generally towards one another from opposite sides, especially diametrically opposite sides, of the first portion of the device.

In some such embodiment forms, each interengagement nose portion may include an obliquely inclined interengagement surface therebeneath, especially an obliquely inclined interengagement surface between the respective nose portion and a radially inner side surface of the respective attachment portion to which the respective nose portion is joined, wherein the angle of inclination of each respective interengagement surface is such that a radially inner end of each respective nose portion is closer to the base portion of the first portion of the device than is a radially outer end of the respective nose portion. Alternatively expressed, in such embodiment forms each interengagement nose portion may comprise a lower interengagement surface, which is that surface directed towards the base portion of the first portion of the device, which forms an angle of less than 90 degrees, e.g. an angle in the range of from about 50 or 60 or 70 to about 80 or 85 or 87 degrees, with a radially inner side surface of the respective attachment portion to which the respective nose portion is joined.

Accordingly, in some such embodiment forms, the respective interengagement nose portions of the pair of attachment portions may form a snap-fit attachment mechanism with the second interengagement means on the head portion of the pedicle screw.

In practising such "snap-fit" embodiments of the anchoring device, for the purpose of bringing the anchoring device and the pedicle screw together and enabling the snap-fit connection between the first and second interengagement means to be made, if desired or necessary a suitable tool may be employed for drawing the two parts together and making the snap-fit attachment connection.

Thus, for use with an anchoring device according to the first aspect of the invention, a pedicle screw may comprise:
a head portion; and
a screw-threaded shaft portion extending from the head portion;
wherein the head portion includes second interengagement means configured for interengagement with the first interengagement means provided on the first portion of the anchoring device, which anchoring device may be an anchoring device according to the first aspect of the invention or any embodiment thereof.

In some example forms of the above-defined pedicle screw the second interengagement means may be provided on, or formed in or on, an outer sidewall portion, especially a radially outer sidewall portion, of the screw head portion. In some embodiments the second interengagement means may comprise one or more, especially at least one pair of oppositely arranged, notches or recesses each for accommodating therein a respective interengagement nose portion of a respective attachment portion extending generally perpendicularly from the base portion of the anchoring device. The pair of notches or recesses of the second interengagement means may be arranged on opposite sides, especially diametrically opposite sides, of the head portion, and facing in opposite radially outward directions from each other.

In some such example forms of pedicle screw each notch or recess of the second interengagement means may include an obliquely inclined interengagement surface therewithin, wherein the angle of inclination of each respective interengagement surface is such that a radially inner end of each respective notch or recess is closer to a lower sidewall portion of the screw head portion than is a radially outer end of the respective notch or recess. Alternatively expressed, in such embodiment forms each interengagement notch or recess may comprise a lower interengagement surface therewithin, which is that surface directed towards the lower sidewall portion of the screw head portion, which forms an angle of less than 90 degrees, e.g. an angle in the range of from about 50 or 60 or 70 to about 80 or 85 or 87 degrees, with a radially inner side surface of the respective notch or recess.

Thus, in such example forms of pedicle screw the internal shape and configuration of each notch or recess of the second interengagement means, or at least the configuration and orientation of the respective interengagement surfaces of the respective notches or recesses of the second interengagement means, may substantially match (or be complementary to) the outer shape and configuration of the respective interengagement nose portions of the respective attachment portions of the first portion of the anchoring device. In this manner, in such embodiments, the respective notches or recesses of the second interengagement means may form a snap-fit attachment mechanism with the respective interengagement nose portions of the pair of attachment portions.

In practical example forms of the above-defined pedicle screw, the upper part of the screw head portion may be configured in any conventional manner, such as in the form of a discontinuous collar, e.g. with a generally cylindrical or tubular shape (which may be of any suitable cross-sectional shape, e.g. circular, elliptical, generally rectangular, or any other suitable shape), with the collar having the respective notches or recesses of the second interengagement means formed in the respective radially outer sidewall potions thereof.

Alternatively or additionally in such practical example forms of the above-defined pedicle screw, the upper part of the screw head portion may be further configured in a conventional manner so as to include a central channel, recess, slot or through-hole therein for accommodating therein a conventional stabilising rod for use in the corrective spinal vertebral surgical operation, and/or to include a screw-threaded locking hole or recess or stub/spigot for having affixed therein or thereto a screw-threaded locking screw or nut for locking such a stabilising rod to the screw head portion - which in either case may only be in example arrangements in which such a direct connection between such a screw and such a stabilising rod is desired to be employed.

In order to facilitate the above-mentioned use of a tool for effecting the drawing together of the screw head portion and the anchoring device to form the snap-fit connection therebetween, the upper sidewall part of the screw head portion may be yet further configured to have one or more, especially a pair of, lateral secondary notches or recesses therein, for enabling such a tool to securely grasp the screw head portion in the drawing together of the two parts and the effecting of the snap-fit connection.

In practical examples, pedicle screws for use with anchoring devices according to the invention may be made from any suitable material, in particular for example from a surgically acceptable metal or metal alloy. Suitable metal alloys may include various titanium alloys (such as an alloy of titanium with one or more other metallic elements selected from the group consisting of: Al, V, Cr, Mn, Fe, Co, Ni, Cu, Zr, Nb, Mo, Sn, Ta, Pd, Ru), cobalt-chromium (CoCr) alloys, and cobalt-chromium-molybdenum (CoCrMo) alloys. Other metal alloys known for use in various surgical implants may also be suitable.

In the anchoring device of the invention, the device comprises a plurality of second portions for anchoring thereto the one or more sub-laminar bands or tapes, with each second portion being configured and arranged for anchoring thereto a said sub-laminar band or tape especially a respective one of a plurality of said sub-laminar bands or tapes. In accordance with the invention the plurality of second portions are arranged equi-angularly or symmetrically around (or radially external to) the first portion of the device. Each such second portion may be integral with the first portion of the device. Furthermore, in some practical example forms each such second portion may be formed together with the first portion of the device as a unitary moulding or casting.

In some such embodiments, the device may comprise at least one pair of second portions, which may be symmetrically arranged extending from opposite, especially diametrically opposite, sides of the first portion of the device and extending in radially opposite directions therefrom. In accordance with the invention, each second portion is configured as an apertured or hollow hoop or loop, especially one with a generally circular internal shape, or as an extension, e.g. a land, flange or tab, extending from the first portion of the device and with an aperture or through-hole formed therein.

Further in accordance with the invention, each of the plurality of second portions of the device is configured so as to lie in a general plane with is substantially co-planar with the corresponding general plane(s) of the other second portion(s) of the device.

In some embodiments of the anchoring device of the invention, the second portions are configured such that the respective general planes of the second portions are substantially co-planar with a general plane of a base portion of the anchoring device from which extend the first interengagement means which provide the interengagement attachment of the first portion of the device to the pedicle screw head portion.

In practical embodiment forms of the anchoring device of the invention, the second portions and the central, first portion may be formed, especially together as a unitary moulding or casting, from any suitable material. Suitable materials may include titanium or a titanium alloy (e.g. an alloy of titanium with one or more other metallic elements selected from the group consisting of: Al, V, Cr, Mn, Fe, Co, Ni, Cu, Zr, Nb, Mo, Sn, Ta, Pd, Ru), various cobalt-based alloys (e.g. alloys of cobalt with chromium and/or molybdenum), as well as various suitably strong and rigid synthetic plastics materials (specific examples of which are already known in the art of synthetic surgical implants).

In general, in practical embodiments, any metal or metallic alloy used to form the anchoring device may desirably be of a material that is chemically compatible with the material from which the associated pedicle screw is formed. By this is meant that the respective materials of the anchoring device and the pedicle screw head portion with which it comes into prolonged contact once in use substantially do not create or lead to any significant mutually-induced corrosion or cold-welding phenomena during their extended use and presence in the spinal cavity of a patient.

In some embodiments of the anchoring device of the invention, each second portion may have mounted therein an insert for providing the means of anchoring to that second portion of the device the or the respective sub-laminar band or tape. The or each insert may be manufactured from any suitable plastics material, for example, although other materials (e.g. a metal or metal alloy) may be possible.

In some such embodiments the or each insert may comprise a central body, in which there is provided a slot or aperture through which passes or is accommodated the or the respective sub-laminar band or tape, and an outer frame configured for mounting the insert in the respective second portion of the device. In such embodiments the outer frame may be configured for being engageable in the respective second portion of the device by means of a snap-fit connection.

In some such embodiments the central body of the or the respective insert may take the form of a short, circular cylinder, which is mounted rotatably, especially freely rotatably, within a geometrically similar circular mounting within the respective frame, so that the orientation of the slot or aperture therein may vary or be self-adjusting so as to adopt an optimum orientation as dictated by the various forces that may be exerted on the anchoring device by the one or more sub-laminar bands or tapes once they have been anchored to the device and tightened into their desired secured (or locked) condition.

In some embodiment forms the slot or aperture within the respective central body of each respective insert may be suitably dimensioned to allow the respective sub-laminar band or tape to pass and slide freely therethrough, in which case it is to be expected that a separate discrete securement or locking device is to be employed elsewhere in the overall arrangement for securing the respective sub-laminar band or tape in its tightened condition once it has been deployed in its desired configuration having been passed or threaded around the relevant vertebra(e) and anchored to the anchoring device.

Alternatively, in other embodiment forms, it may be possible for the or each respective slot or aperture within the respective central body of the respective insert to itself include a suitable securement or locking device which allows the respective sub-laminar band or tape to be secured directly within the respective insert of the device. By way of example, a suitable form of such integrated securement or locking device may comprise a one-way-only clamping mechanism, which allows a band or tape to slidingly pass through the slot or aperture in one direction only, e.g. for tightening purposes, yet to be restrained by gripping and prevented from being drawn back through the slot or aperture in the opposite direction.

For use in or with embodiments of the anchoring device of the invention, any suitable known form(s) of sub-laminar bands or tapes may be used, examples of which are well-known in the art. Such sub-laminar bands or tapes may for instance be formed of a non-elastic poly-filament woven or braided material, e.g. of a suitable synthetic plastics or polymeric material (such as polyester terephthalate, among others).

In some alternative embodiments of the anchoring device of the invention, the construction and configuration of its first and second portions may be somewhat simplified, in particular such that the device is formed as a substantially unitary one-piece structure, especially with no incorporated moving parts, and which may be more readily deployed in combination with a simpler modified form of pedicle screw head or even with one or more known designs of pedicle screw head. Such a simpler design of anchoring device may also be cheaper to manufacture.

Thus, in some alternative embodiments of the anchoring device of the invention, instead of the anchoring device and the head portion of the pedicle screw being attachable and unitable by virtue of a snap-fit connection (such as by use of the above-defined first and second interengagement means and the device being brought together with the pedicle screw head portion from below by virtue of the pedicle screw's threaded shaft being inserted through the hole or aperture in the first portion of the device from thereabove), in some alternative embodiments of the invention in its various aspects the anchoring device and the head portion of the pedicle screw may be attachable and unitable by virtue of a push-fit or friction-fit or mere abutment-fit connection, with the anchoring device being able to be brought together with the pedicle screw head portion from above by virtue of the hole or aperture in the first portion of the device being placeable over the pedicle screw's head portion from thereabove and the two parts united by means of alternatively constructed first and second interengagement means on the respective anchoring device and pedicle screw head portion.

Accordingly, in some alternative embodiments of the anchoring device of the invention, the first portion of the device may be configured such that it is attachable to and unitable with the head portion of the pedicle screw, via the interengagement therewith of the first interengagement means, by means of placement of a hole or aperture in the first portion of the device over the pedicle screw's head portion from thereabove, i.e. from the side thereof opposite to that from which extends the pedicle screw's threaded shaft.

In some such alternative embodiments of the anchoring device, the first portion of the device may still or again be configured for attachment to the head portion of the pedicle screw by interengagement of the device's first interengagement means with at least an outer sidewall portion, especially a radially outer sidewall portion, of the screw head portion, but now the first interengagement means may comprise one or more, especially at least one pair of oppositely or diametrically oppositely arranged, flanged or seating or bearing portions, which may be integral with, or formed as extensions of or by portions of, the first portion of the device.

For use in combination with such an alternative embodiment anchoring device, in an alternative form of pedicle screw the head portion of the pedicle screw may still or again include second interengagement means which are provided on, or formed in or on, an outer sidewall portion, especially a radially outer sidewall portion, of the screw head portion, but now the second interengagement means may comprise one or more, especially a circumferential or alternatively at least one pair of oppositely or diametrically oppositely arranged, externally extending flanges, lips, steps or lands, or possibly even recesses, recessed lands or indentations or shoulders, formed on or in the outer sidewall of the pedicle screw head portion, with which may engage by simple mechanical abutment or seating or bearing the respective corresponding flanged or seating or bearing portion(s) constituting the first interengagement means of the anchoring device.

Thus, also for use with an anchoring device according to the first aspect of the invention, an alternative form of pedicle screw may comprise:
a head portion; and
a screw-threaded shaft portion extending from the head portion;
wherein the head portion includes second interengagement means configured for interengagement with the first interengagement means provided on the first portion of the anchoring device, which anchoring device may be an anchoring device according to the first aspect of the invention or any embodiment thereof,
and wherein the second interengagement means are provided on, or formed in or on, an outer sidewall portion, especially a radially outer sidewall portion, of the screw head portion;
but now the second interengagement means comprise one or more, especially a circumferential or alternatively at least one pair of oppositely or diametrically oppositely arranged, externally extending flanges, lips, steps or lands, formed on the outer sidewall of the pedicle screw head portion, especially with which may engage by simple mechanical abutment or seating or bearing the or the respective first interengagement means of the anchoring device.

In many practical forms of such alternative embodiments of anchoring device and forms of pedicle screw the respective one or more (especially plurality of) flanged or seating or bearing portions constituting the first interengagement means of the anchoring device and the one or more external flanges, lips, steps or lands, or recesses, recessed lands or indentations or shoulders, constituting the second interengagement means of the pedicle screw head portion, may be shaped, dimensioned and positioned relative to each other such that they substantially match or approximate in configuration or fit together with one another in respective pairs thereof such that they may readily unite with each other in a simple abutment or seating or bearing manner, and thus so that the anchoring device and the pedicle screw head portion may be readily unitable by virtue of a simple mechanical slide-over or push-fit or friction-fit connection.

In some such alternative embodiments of anchoring device the device may be formed as a substantially unitary one-piece structure, e.g. by any suitable moulding technique, optionally with cutting out or drilling of any appropriate holes, apertures, slots or slits that may be required for forming other features of the device.

Such one-piece embodiment forms of anchoring device may, as for other embodiment devices, be formed from any suitable material, such as titanium or a titanium alloy (e.g. an alloy of titanium with one or more other metallic elements selected from the group consisting of: Al, V, Cr, Mn, Fe, Co, Ni, Cu, Zr, Nb, Mo, Sn, Ta, Pd, Ru), various cobalt-based alloys (e.g. alloys of cobalt with chromium and/or molybdenum), or various suitably strong and rigid synthetic plastics materials, e.g. polyether ether ketone "PEEK" or similar material, to name just one example thereof amongst many possible alternatives.

Such one-piece embodiment forms of anchoring device may be substantially flat or planar, or alternatively non-flat or non-planar, in general cross-sectional shape. In the case of non-flat/non-planar forms, the anchoring device may be configured for example with at least one portion thereof curved or bent or concave (or convex) or ramped or stepped with respect to the remainder of the device. Such a curved or bent or ramped or stepped portion may for instance be constituted by one of the flanged or seating or bearing portions constituting the first interengagement means of the device.

In some such alternative embodiments of anchoring device, especially those in which the device is formed as a substantially one-piece structure, the construction and configuration of the second portions (for anchoring the sub-laminar band(s) or tape(s)) may also be somewhat simplified. Accordingly, instead of distinct hoops or loops forming the second portions, optionally with a respective insert mounted in a frame portion thereof and including a slot or aperture through which is passable and securable a respective sub-laminar band or tape, each second portion of the device may comprise one or more, or a plurality of, holes or apertures or slots or slits formed in and/or extending through the material of the respective second portion.

Such second portions of the device, especially a pair of said second portions, containing the said holes/apertures/slots/slits may be arranged or located to either side of the first portion of the device, and especially in the case of a pair of oppositely or diametrically oppositely arranged flanged or seating or bearing portions forming the first engagement means, respective ones of the pair of said second portions may be arranged or located intermediate of or between respective ones of those flanged or seating or bearing portions forming the first engagement means. Each second portion may comprise any number of (especially one or more of or a plurality of) holes/apertures and/or any number of (especially one or more of or a plurality of) slots or slits in any combination or spatial arrangement or distribution, in order to achieve any desired or appropriate anchoring or securing of the respective band or tape passed therethrough. For example, certain one(s) of any such hole(s)/aperture(s) or slots/slits may more appropriately serve a tightening or tensioning function, whereas certain other one(s) of any such hole(s)/aperture(s) or slots/slits may more appropriately serve a securing or locking function.

If desired or necessary, any final anchoring or securement of a respective sub-laminar band or tape in any suitable one (or more) of such hole(s)/aperture(s) or slots/slits may be effected or facilitated by insertion thereinto of a suitable locking element, such as a locking screw, plug or like insertable clamping or locking element, optionally in combination with a suitable grommet, collar or similar anchoring insert. This feature may also apply to the practical deployment of the first embodiment anchoring devices as discussed hereinabove.

In certain ones of such alternative embodiment forms, the defining perimeter(s) of any one or more of such hole(s)/aperture(s) or (especially) slots/slits may be wholly contained within the material of the relevant second portion of the device in which it/they is/are formed. However, in certain other ones of such alternative embodiment forms, the defining perimeter(s) of any one or (especially) two or more of such hole(s)/aperture(s) or (especially) slots/slits may be only partially contained within the material of the relevant second portion of the device in which they are formed, so that the relevant hole(s)/aperture(s) or (especially) slots/slits is/are open and unbounded on at least one side or portion thereof, especially on a radially inner side or portion thereof, thereby creating a (or a respective) tab or tongue inbetween at least one pair thereof. This tab/tongue feature may serve to assist the insertion and threading through the slots/slits of the respective sub-laminar bands or tapes, and/or may also serve as a deformable or displaceable member that forms its own interference fit against an outer sidewall portion of the pedicle screw head once it has been inserted into the central hole/aperture of the device upon uniting of the two components together.

Some practical examples of some useful tightening/tensioning and securing/locking arrangements of bands or tapes passed through various hole(s)/aperture(s) and slots/slits in such second portions of such alternative embodiment devices will be described further hereinbelow in the context of some specifically described practical example embodiments of the invention as illustrated in the accompanying drawings.

Within the scope of this application it is expressly intended that the various aspects, embodiments, examples and alternatives set out in the preceding paragraphs, in the claims and/or in the following description and drawings, and in particular the individual features thereof, may be taken independently or in any combination. Thus, any one or more features referred to or described with reference to one particular embodiment should be construed as being applicable to any or all embodiments, unless expressly stated otherwise or such features are incompatible.

### BRIEF DESCRIPTION OF THE DRAWINGS

Various embodiments of the various aspects of the present invention will now be described in detail, by way of non-limiting example only, with reference to the accompanying drawings, in which:
FIGURE 1 is an illustrative posterior view of a human spinal region exposed during spinal deformity corrective surgery, showing an example of a pair of rigid constructs formed by a pair of stabilising rods each affixed by a series of screws to the various thoracic vertebrae;
FIGURES 2(a) and (b) are, respectively, a posterior and a left-side x-ray image of the spinal region of FIG. 1, illustrating the typical three-dimensional arrangement of the various screws and rod constructs;
FIGURE 3 is a perspective view of another example arrangement, prior to deployment in a patient, of a stabilising rod construct which has had attached thereto, and locked thereon (by locking screws), a series of pedicle screws via their "tulip-heads";
FIGURE 4 is a posterior view of an anchoring device according to one embodiment of the present invention, showing the device *in situ* mounted on a pedicle screw with a pair of sub-laminar bands or tapes anchored to the device, the device ready for uniting with a stabilising rod in a spinal deviation corrective surgical procedure;
FIGURES 5(a) and (b) are, respectively, a frontal view of a modified pedicle screw and an end-on view of an anchoring device according to an embodiment of the first aspect of the present invention, for use together in forming an anchoring arrangement of another aspect of the invention;
FIGURE 6(a) is a frontal view of just the modified pedicle screw shown in FIG. 5(a);
FIGURE 6(b) is a side-on view of the head portion of the pedicle screw of FIG. 6(a);
FIGURE 7(a) is an end-on view of just the anchoring device show in FIG. 5(b);
FIGURE 7(b) is a side-on view of the anchoring device of FIGS. 5(b) and 7(a);
FIGURE 7(c) is the same view of the same embodiment anchoring device as in FIG. 7(b), but showing the manner in which a sub-laminar band or tape is passed through one of the inserts located within one of the hoop-like second portions of the device;
FIGURE 7(d) is a top plan view of the anchoring device of FIGS. 5(b) and 7(a)-7(c);
FIGURES 8(a) and (b) are, respectively, a top plan view and side-on view of a central body of an insert for insertion into each of the hoop-like second portions of the anchoring device of FIGS. 7(a)-7(d);
FIGURE 9(a) is a frontal view of an alternative form of modified pedicle screw for use with an alternative embodiment of anchoring device according to the invention, in which a push-fit rather than a snap-fit attachment mechanism is employed to unite the two components together;
FIGURE 9(b) is a side-on view of the head portion of the alternative modified pedicle screw of FIG. 9(a);
FIGURE 10 is a perspective view of an alternative embodiment of anchoring device according to the invention, which is usable in combination with various designs of pedicle screw, including the modified design thereof shown in FIGS. 9(a) & 9(b) but also other known designs such as that shown in FIGS. 12(a) & (b);
FIGURE 11 is a front elevational view of the alternative embodiment anchoring device of FIG. 10;
FIGURE 12(a) is a perspective view of the head portion of one known design of pedicle screw with which the alternative embodiment anchoring device of FIG. 10 may also be used;
FIGURE 12(b) is a side elevational view of the known pedicle screw head portion of FIG. 12(a);
FIGURE 13 is a perspective view of the combination of the alternative embodiment anchoring device of FIG. 10 together with the known pedicle screw of FIGS. 12(a) & (b), showing them attached and united together;
FIGURE 14 is a top plan view of the attached combination of FIG. 13, together with a locking screw anchoring a stabilising rod extending through the pedicle screw head, showing the combination ready for receiving various sub-laminar bands or tapes passing through the various apertures and/or slots/slits thereof;
FIGURE 15 is a simplified schematic illustration showing one example manner in which a sub-laminar band or tape can be passed through the various apertures and slots/slits of the anchoring device arrangement of FIG. 14 and secured in place therein in order to anchor the band or tape with respect to a spinal vertebral lamina during corrective spinal vertebral surgery;
FIGURE 16 is a top plan view of a slightly modified embodiment of anchoring device according to the invention, which is very similar to that of FIGS. 10, 11, 13 & 14 but with a slightly different configuration of band/tape anchoring/securement slots;
FIGURE 17 is a simplified schematic illustration of another example manner in which a sub-laminar band or tape can be passed through the various apertures of the anchoring device arrangement of FIGS. 14 or 16 and secured in place therein in order to anchor the band or tape with respect to a spinal vertebral lamina during corrective spinal vertebral surgery; and
FIGURE 18 is a simplified schematic illustration of yet another example manner in which a sub-laminar band or tape can be passed through the various apertures of the anchoring device arrangement of FIGS. 14 or 16 and secured in place therein in order to anchor the band or tape with respect to a spinal vertebral lamina during corrective spinal vertebral surgery.

### DETAILED DESCRIPTION OF EMBODIMENTS

In the accompanying drawings, any specific dimensions (e.g. in mm or cm) that may be annotated thereon are to be considered to be non-limiting and for purely illustrative example purposes only.

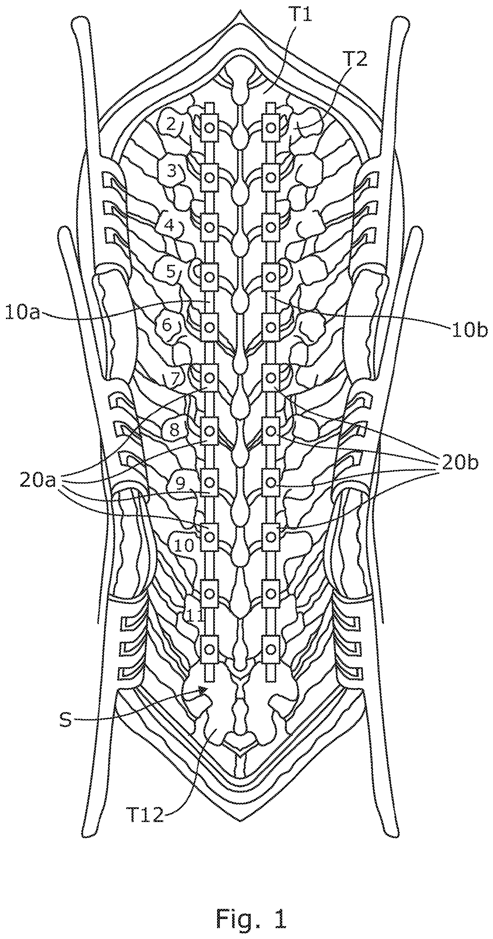

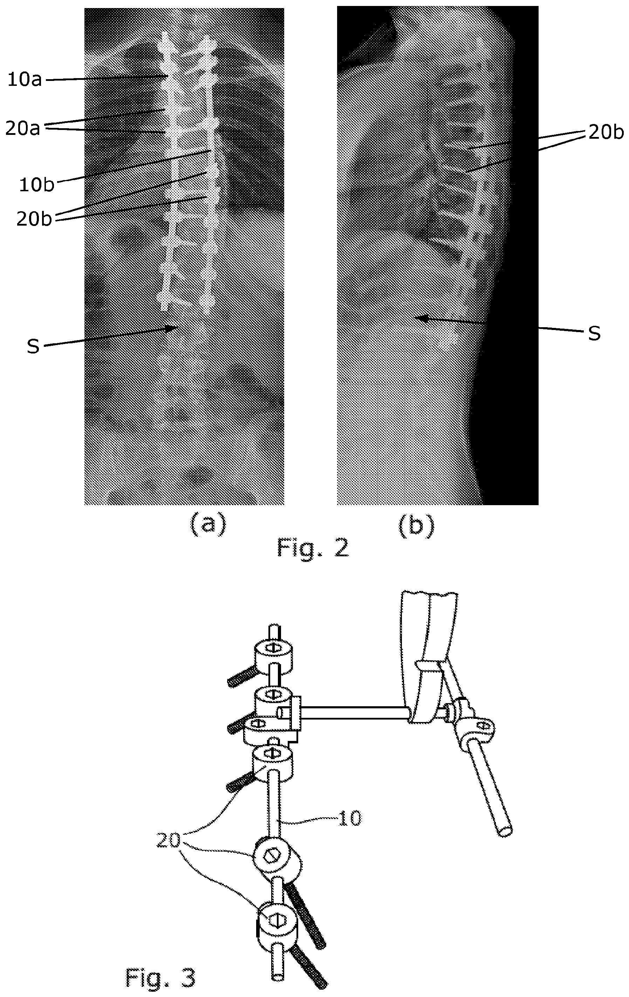

Referring firstly to FIG. 1, this shows purely by way of an illustrative example a human spinal region, in posterior view, which has been exposed during spinal deformity corrective surgery to mount a pair of substantially rigid constructs or assemblies onto the various thoracic vertebrae (labelled conventionally as T1 - T12) for the purpose of correcting a spinal deformity such as that caused by scoliosis. In this example, a pair of generally parallel stabilising rods 10a, 10b have been mounted on and secured to a respective series of screws 20a, 20b each of which has been inserted into the bone of a respective pedicle or other part of the relevant vertebra of the spinal column S. FIGS. 2(a) and (b) show the overall spinal implant arrangement more clearly in three dimensions in the form of x-ray images. (It is to be understood, of course, that the arrangement shown in FIGS. 1 and 2 of two elongate stabilising rods 10 and their associated screws being affixed into each of the thoracic vertebrae T1 - T12 may well be an extreme example, and it is possible for any shorter or different arrangement of one or any plurality of stabilising rods 10 and any suitable number of attachment/affixation screws 20, e.g. pedicle screws, to be provided for creating any desired size, layout and configurational arrangement for use in anchoring one or more sub-laminar bands or tapes with respective to any one or more of the spinal vertebrae.

In practice, the function of each of the stabilising rods 10a, 10b of FIG. 1 may typically be supplemented by any suitable number and arrangement of one or more sub-laminar bands or tapes (not shown in FIG. 1), which pass around appropriate portions of the relevant vertebrae and are anchored to the respective rod 10 to provide a means of pulling the spinal column S into the desired corrective configuration and holding it there upon securement or locking of the respective sub-laminar bands or tapes in their tightened state. Typically these steps will be carried out after the final insertion of the screws into the relevant vertebrae pedicles and the accompanying final anchoring and locking (using conventional locking screws/nuts) of the relevant rod onto the heads of the relevant pedicle screws. In this manner the final arrangement enables the necessary corrective relative configuration of each of the relevant vertebrae with respect to the relevant stabilising rod 10 to be assured and maintained. It is generally necessary of course that suitable anchoring points in the arrangement are provided for the relevant sub-laminar bands or tapes so they can exert their corrective torsional and other forces, and these anchoring points can be most usefully provided by the heads of the pedicle screws 20a, 20b themselves. It is thus to the means of anchoring such sub-laminar bands or tapes with respect to the screws 20a, 20b that embodiments of the invention in its various aspects are especially directed.

FIG. 3 shows more clearly a representative example of an arrangement, before being deployed in a patient, of a stabilising rod with a series of associated pedicle screws locked thereon (by conventional locking screws) via their "tulip-heads".

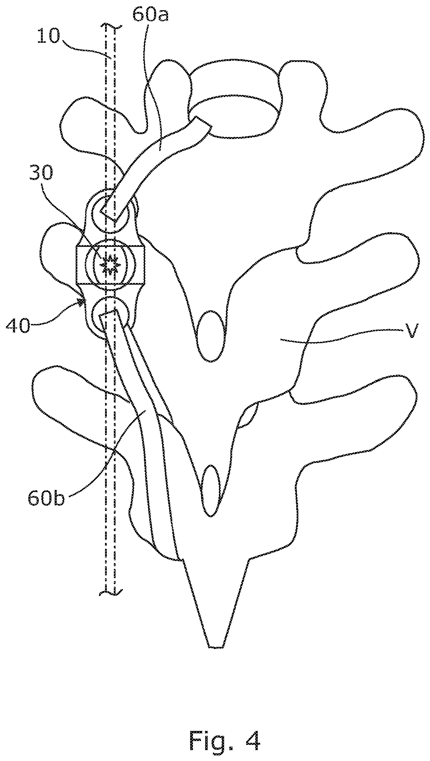

Turning now to FIGS. 4 to 7, these various FIGS. show the principal components of an embodiment of an anchoring arrangement useful for anchoring one or more sub-laminar bands or tapes with respect to one or more pedicle screws. The arrangement comprises in particular an anchoring device 40, together with a modified pedicle screw 30 (which is to say a pedicle screw with a modified construction or configuration in comparison with known pedicle screws hitherto known and used in the art), which are unitable together in a novel fashion as a result of their unique and novel constructions.

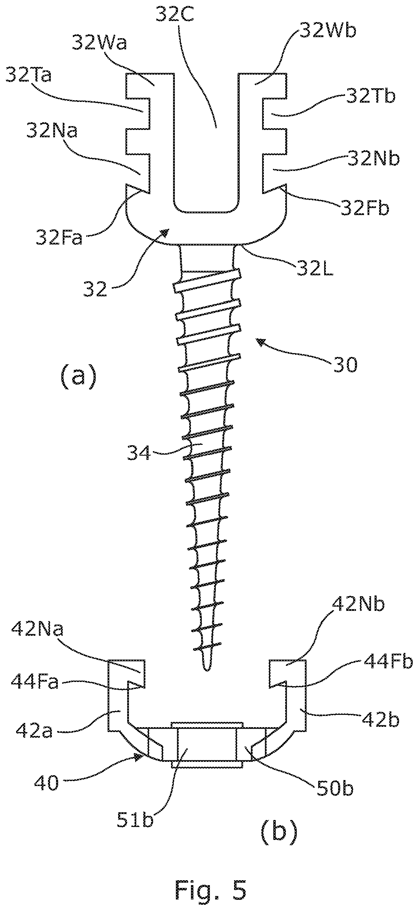

As shown in FIG. 4, the anchoring device 40 is shown here *in situ* having been mounted on the underside or base of (i.e. at least in the region of the lower sidewall portion 32L of) the head portion 32 (see FIG. 5) of a modified pedicle screw 30 with a pair of sub-laminar bands or tapes 60a, 60b anchored to the device 40, the device 40 being ready for uniting with a stabilising rod 10 (shown in phantom lines for clarity) in a spinal deviation corrective surgical procedure. The sub-laminar bands or tapes 60a, 60b may be formed of a conventional material, e.g. a non-elastic poly-filament woven or braided material such as of polyester terephthalate or other suitable synthetic plastics or polymeric material. The pedicle screw 30 has been inserted in a known manner into the bone of the pedicle of the relevant vertebra V which is that spinal segment which is to be fixed into a new position in the surgical correction procedure. The anchoring device 40, as well as being securely attached to the head portion 32 of the inserted pedicle screw 30 via their novel respective interengageable features (as described further below), serves as an anchor for anchoring, relative to the screw 30, each of the pair of sub-laminar bands or tapes 60a, 60b, each of which passes round a respective vertebral lamina of the selected vertebra V, thereby enabling the bands/tapes 60a, 60b to be used more efficiently and reliably to apply the necessary forces to effect the desired deformity correction.

Indeed, the application of the sub-laminar bands or tapes 60a, 60b to the adjacent vertebrae above and below the vertebra holding the pedicle screw 30 allows fixation of up to three apical segments with just one effective anchoring arrangement and two bands/tapes. In particular, this novel arrangement may equate to a more efficient use of space, thereby ameliorating the problem of screw-head crowding in the region of the apex of the spinal concavity, which can often present practical difficulties in the deployment of known spinal implants used in known spinal corrective surgical procedures.

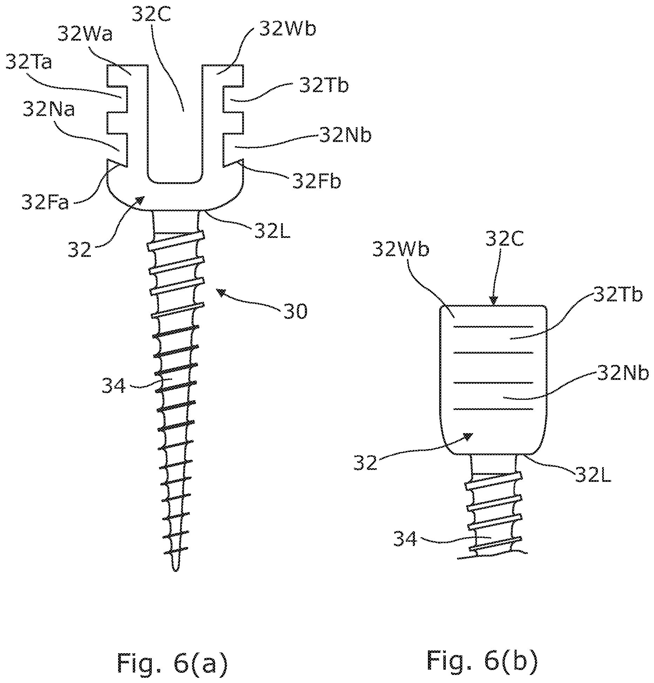

FIGS. 5(a) and 6(a) & 6(b) show the construction and configuration of one modified pedicle screw 30 in accordance with an embodiment of one aspect of the present invention. The screw, e.g. formed of a conventional titanium alloy, comprises a lower shaft portion 34, which is formed with a conventional screw-thread for insertion (optionally in a pre-drilled hole in the bone) into a selected pedicle or other portion of a selected vertebra, and an upper head portion 32 which is configured to fulfil a dual role - namely to provide a means of attachment to a stabilising rod 10 as well as to provide a site of anchoring for one or more sub-laminar bands or tapes 60a, 60b. The head portion 32 is formed with an internal channel 32C therein which is sized and configured to accommodate therein a stabilising rod 10, which rod 10 may be locked in the channel 32C in a conventional manner as or when desired by insertion therein of a known form of locking screw (the internal sidewalls of the channel 32C are shown in FIG 5 as being without any internally screw-thread, for the sake of clarity, although it is to be understood that any suitable internal screw-threading may be employed for this rod-locking purpose. Owing to the configuration of the internal channel 32C, the remnant sidewall portions of the screw head portion 32 may be thought of as being constituted by a pair of diametrically opposed upstanding wall portions 32Wa, 32Wb.

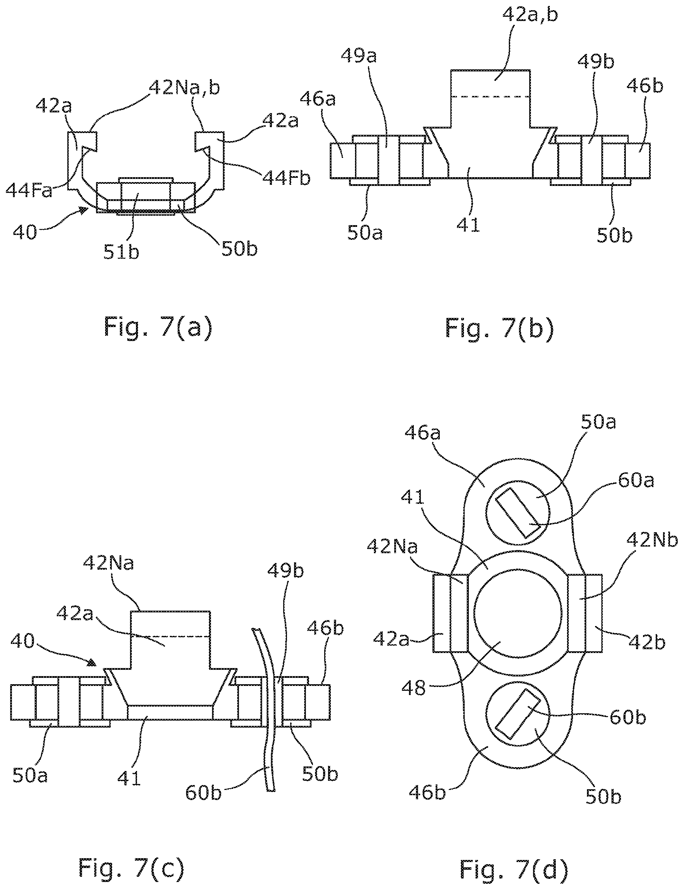

The head portion 32 of the screw 30 is provided on each respective outer sidewall of each of the upstanding wall portions 32Wa, 32Wb with a respective interengagement notch or recess 32Na, 32Nb, each of which is configured to interengage with a respective interengagement detent provided on the first portion of the anchoring device 40 (as described further below). Each interengagement notch or recess 32Na, 32Nb is formed with an obliquely inclined floor surface 32Fa, 32Fb. The angle of inclination of each respective floor surface 32Fa, 32Fb - which may typically be less than 90 degrees, e.g. an angle in the range of from about 50 or 60 or 70 up to about 80 or 85 or 87 degrees, relative to a radially inner side surface of the respective notch or recess 32Na, 32Nb - is therefore such that a radially inner end of each respective notch or recess 32Na, 32Nb is closer to a lower sidewall portion 32L of the screw head portion 32 than is a radially outer end of the respective notch or recess 32Na, 32Nb. Thus, this shape of the lower (i.e. floor) region of each notch or recess 32Na, 32Nb enables it to more assuredly and securely retain therein a respective interengagement detent 42a, 42b (FIG. 7(a)) provided on the central portion of the anchoring device 40 once it has been interengaged therewith.

In order to facilitate the use of a conventional reduction device or other tool for effecting the drawing together of the screw head portion 32 and the anchoring device 40 to form the snap-fit connection therebetween, the upper sidewall part of the screw head portion 32 is further provided with a pair of lateral, straight-walled secondary notches or recesses 32Ta, 32Tb therein, for enabling such a device/tool to securely grasp the screw head portion 32 in the drawing together of the two parts and the effecting of the snap-fit connection.

FIGS. 5(b) and 7(a), 7(b), 7(c) & 7(d) show the construction and configuration of the anchoring device 40 which is in accordance with an embodiment of a primary aspect of the present invention. The anchoring device 40 comprises a central first portion 41, which includes a lower generally cylindrical or annular or toroidal base portion thereof which, once the anchoring device 40 has been interengaged with the screw head portion 32 by means of the respective first and second interengagement snap-fit features (as described further below), substantially abuts the lower sidewall portion 32L of the screw head portion 32. The base portion is apertured or hollow, e.g. with a suitably sized circular hole or aperture 48 therein for allowing passage (in a relatively downward direction, as shown in the drawing) therethrough of the screw-threaded shaft 34 of the pedicle screw 30 during the attachment (in a relatively upward direction, as shown in the drawing) of the anchoring device 40 thereto.

Extending or protruding upwardly from, and especially perpendicularly upwardly from, opposite sides of the base portion of the central portion 41 of the device 40 are a pair of mutually facing attachment portions 42a, 42b, which take the form of a pair of diametrically oppositely arranged, and generally mutually parallel, protruding lugs, tabs or arms 42a, 42b. Each attachment portion 42a, 42b terminates at a distal end thereof in a respective radially inwardly projecting interengagement nose portion 42Na, 42Nb, whereby the interengagement nose portions 42Na, 42Nb point generally towards one another from diametrically opposite sides of the central portion 41 of the device 40.

Each interengagement nose portion 42Na, 42Nb includes an obliquely inclined interengagement floor surface 42Fa, 44Fb therebeneath, the angle of inclination of each of which substantially matches (or is complementary to) the corresponding angle of inclination of the respective one of the floor surfaces 32Fa, 32Fb of the notches or recesses 32Na, 32Nb in the outer sidewall of the head portion 32 of the screw 30. The pair of interengagement nose portions 42Na, 42Nb on the anchoring device 40 are thus configured for interengagement with the respective notches or recesses 32Na, 32Nb in the outer sidewall of the screw head portion 32 via the abutting and mating together of the respective pairs of interengaging floor surfaces 32Fa, 42Fa and 32Fb, 44Fb on the two components 32, 40, once they have been brought together into their mutually attached relationship through the resulting "snap-fit" mechanism.

For the purpose of bringing the anchoring device 40 and the pedicle screw 30 together and enabling the snap-fit connection between the respective pairs of interengagement nose portions and notches or recesses 42Na, 32Na and 42Nb, 32Nb to be made, if desired or necessary a suitable tool may be employed for drawing the two components together and making the snap-fit attachment connection. A conventional reduction tool or any other suitable tool or implement may be used for this purpose, for example.

For the purpose of anchoring the one or more sub-laminar bands or tapes 60a, 60b to the anchoring device 40, a pair of generally circular aperture hoop- or loop-like extensions 46a, 46b, e.g. in the form of apertured lands, flanges or tabs, are provided, which extend general radially outwardly from the central portion 41 of the device and may be formed integrally therewith. The pair of apertured extensions 46a, 46b are arranged equi-angularly around, i.e. diametrically opposite one another on opposite sides of, the central portion 41 of the device 40, and they lie generally in the same plane as the general plane of the central portion 41.

The main elements of the anchoring device 40, which is to say at least the pair of apertured extensions 46a, 46b, the central portion 41 and the attachment portions 42a, 42b, are formed together as a unitary moulding or casting, e.g. of a titanium alloy or a suitably strong and rigid synthetic plastic material. In general, however, the material of the anchoring device 40 should be one which is chemically compatible with the material from which the associated pedicle screw 30 is formed, so that the respective materials from which these components are made do not, once they come into prolonged contact with other over time, do not create or lead to any significant mutually-induced corrosion or cold-welding phenomena during their extended use and presence in the spinal cavity of a patient.

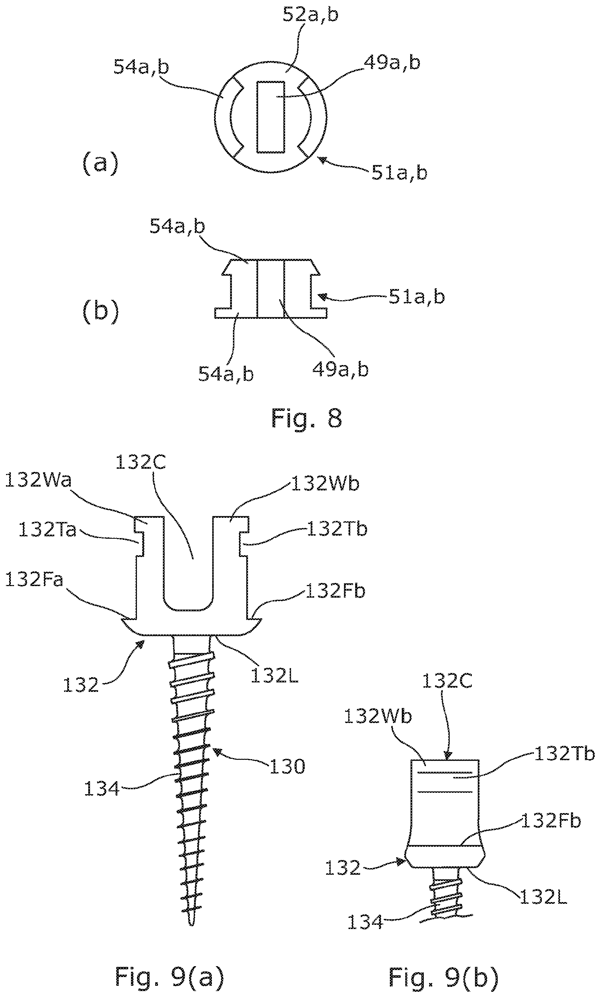

As shown in FIGS. 7(a), 7(b), 7(c), 7(d) and 8(a) & (b), each of the apertured extensions 46a, 46b has mounted within it a respective insert 50a, 50b, e.g. made from a suitable synthetic plastics material, which inserts 50a, 50b provide the necessary anchoring locations for the anchoring to the device 40 of the respective sub-laminar bands or tapes 60a, 60b.

Each insert 50a, 50b comprises a central body 51a, 51b (e.g. in the form of a short, circular cylinder) in which there is provided an axially-extending slot or aperture 49a, 49b through which is able to be passed or accommodated the respective sub-laminar band or tape 60a, 60b. Each central body 51a, 51b is mounted in a respective outer frame member (e.g. comprising a pair of upper and lower annular plates which carry the central body 51a, 51b therebetween) which is itself mounted in the respective apertured extension 46a, 46b. Each outer frame member of each respective insert 50a, 50b is mounted in the respective apertured extension 46a, 46b by means of a snap-fit connection.

The central body 51a, 51b of each insert 50a, 50b is mounted in its respective frame member in such a way that it is freely rotatable within its frame member so that the orientation of the respective slot or aperture 49a, 49b therein may be variably self-adjusting so as to adopt an optimum orientation as dictated by the various forces that may be exerted on the respective insert 50a, 50b (and the anchoring device 40 generally) by the one or more sub-laminar bands or tapes 60a, 60b once they have been anchored to the device 40 and tightened into their desired secured (or locked) condition.

As shown in FIGS. 8(a) & (b), each central body 51a, 51b of each insert 50a, 50b is formed as a short, circular cylinder, and comprises a respective solid circumferential rim 52a, 52b on one surface thereof and an incomplete circumferential ring 54a, 54b on the opposite surface thereof, in order to permit a secure placement (e.g. in the manner of a bayonet-type fixing) of the respective central body 51a, 51b in its respective frame member.

In the embodiment shown in the FIGS., each of the slots or apertures 49a, 49b within the respective central bodies 51a, 51b of the inserts 50a, 50b is dimensioned to allow the respective sub-laminar band or tape 60a, 60b to pass and slide freely therethrough, in order to optimise the tightening and final positioning capability of the anchoring device 40. However, in this case it will then generally be required that a separate discrete securement or locking device (not shown) is employed elsewhere in the overall arrangement for securing the respective sub-laminar bands or tapes 60a, 60b in their tightened conditions once they have been deployed in their desired configurations having been passed or threaded around the relevant vertebra(e) and anchored to the anchoring device 40. Any conventional form of securement or locking device, examples of which are widely used available in the art, may be used for this purpose.

Alternatively, in other embodiment forms, it may be possible for the or each respective slot or aperture 49a, 49b within the respective central body 51a, 51b of the respective insert 50a, 50b to itself include a suitable securement or locking device which allows the respective sub-laminar band or tape 60a, 60b to be secured directly within the respective insert 50a, 50b of the device 40. Again, practical examples of suitable such one-way-only clamping mechanisms, which allows a band or tape 60a, 60b to slidingly pass through the slot or aperture 49a, 49b in one direction only, e.g. for tightening purposes, yet to be restrained by gripping and prevented from being drawn back through the slot or aperture 49a, 49b in the opposite direction, are well-known and available in the art.

Looked at in more general terms, the anchoring arrangement of the illustrated embodiment of the invention - especially as it is illustrated in FIG. 4 - increases the pull-out strength of the screw 30 by sharing the load exerted by the sub-laminar bands or tapes 60a, 60b over three spinal segments, allowing greater translational forces to be applied during the deformity correction, as well as a degree of direct vertebral de-rotation at the spinal apex.

Furthermore, using this configuration of anchoring arrangement, the head portion 32 of the screw 30 may still be manipulated and translated towards a stabilising rod 10 if desired or appropriate, and it may also if desired be used to de-rotate the vertebra using conventional reduction tools (examples of which are well-known in the art).

In the formation of the arrangement during a surgical procedure, once the screw 30 is positioned and the bands/tapes 60a, 60b secured, the screw 30 can then be translated to the rod 10, benefiting from superior pull-out strength, the ability to derotate and potentially to control up to three apical vertebrae with one screw 30, thereby ameliorating the problem of screw-head crowding in the apex of the spinal concavity.

The pedicle screw 30 has been modified, in comparison with a conventional screw, in such a way that the screw "tulip head" 32 can receive the anchoring device 40 which permits the attachments of the sub-laminar bands 60a, 60b, etc to the screw 30. The device 40 is applied to the base of the screw head 32, in a similar manner to a washer, and is self-secured thereon by a snap-on mechanism. Once the anchoring device 40 is attached, and the bands/tapes 60a, 60b, etc have been secured to the device 40 and the relevant vertebrae, the screw 30 may then be manipulated directly to the rod 10, allowing translation and de-rotation of up to three segments of the spine per screw 30 onto the rod 10 using a reduction tool that is attached to the tulip head 32.

The sub-laminar bands/tapes 60a, 60b, etc are positioned under the chosen laminae and looped into the anchoring hoops/loops 46a, 46b of the anchoring device 40. The pedicle screw tulip head 32 has been modified with a novel pair of notches 32Na, 32Nb at the base 32L of the head 32. These notches 32Na, 32Nb permit the afore-mentioned snap-on attachment of the device 40 to the screw head 32. The device 40 is locked on using a reduction tool that stabilizes the tulip head 32 (via the upper manipulation notches 32Ta, 32Tb) and draws the device 40 onto the screw head 32 until an audible "snap-on" is heard.

Accordingly, by way of practical example only, a summary of the sequence of principal steps used in the deployment of the particular embodiment anchoring arrangement described above may be as follows:
1. The pedicle screw hole is prepared to receive a screw in a standard fashion.
2. Sub-laminar bands 60a, 60b are positioned beneath the chosen laminae, usually those above and below the level of the screw 30.
3. The bands 60a, 60b are looped through the anchoring device 40, which can receive two bands 60a, 60b. Redundant lengths of the bands may be maintained.
4. The device 40 is placed over the shaft 34 of the screw 30 before the screw is advanced into the pedicle.
5. Once the screw 30 is situated, the device 40 is definitively reduced onto the pedicle screw head 32 using a reduction device/tool.
6. The bands 60a, 60b are then tensioned between the bone and the device 40 and secured/locked in place.
7. The rod reduction tool is then used to translate the screw head 32 (and spine with it) to the rod 10.
8. De-rotation of the spine may then be attempted.
9. A locking screw is then tightened onto the screw head 32, thereby securing the rod 10 thereto.

Although one particular embodiment anchoring arrangement has been described above, and illustrated in the drawings referred to, in terms of an anchoring arrangement which comprises a single anchoring device 40 + pedicle screw 30 combination, together with a pair of sub-laminar bands or tapes 60a, 60b, it is to be understood that other anchoring arrangements also within the scope of the invention may be devised which include any desired or appropriate other numbers of anchoring device + pedicle screw combinations in conjunction with any desired or appropriate number(s) of associated sub-laminar bands or tapes.

Turning now to FIGS. 9 to 18, these FIGS. illustrate an alternative embodiment form of anchoring device 140 within the scope of the invention, which may be used in combination with either an alternative form of modified pedicle screw 130 or alternatively still in combination with a known design of pedicle screw 230.

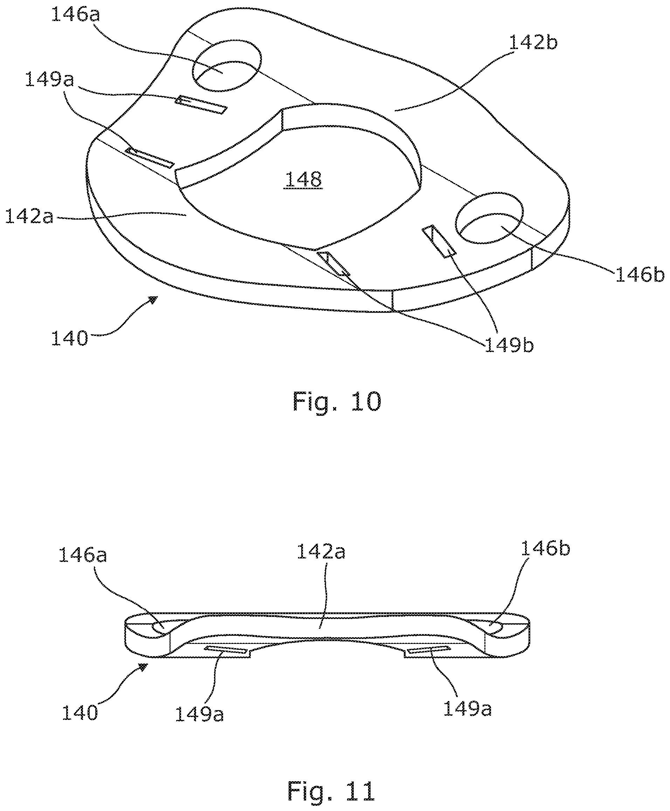

FIGS. 9(a) and 9(b) illustrate an alternative form of modified pedicle screw 130 for use with an alternative embodiment form of anchoring device 140 as shown in FIGS. 10 and 11. These two components 130, 140 are unitable together by means of a mechanically simpler push-fit abutment-type connection, rather than a snap-fit attachment mechanism relying on interengaging teeth-like features as in the first embodiment's components described above in relation to FIGS. 5 to 7.

As shown in FIGS. 10 and 11, the alternative form of anchoring device 140 is formed as a unitary one-piece structure, e.g. moulded from a titanium alloy or alternatively a suitable polymer material, such as PEEK (polyether ether ketone) or similar. The first, main body portion of the device 140 comprises a central hole or aperture 148 which is placeable over the head portion 132 of the pedicle screw 130 (see FIGS. 9(a) & 9(b)) from thereabove, i.e. from the side thereof opposite to that from which extends the pedicle screw's threaded shaft 134.

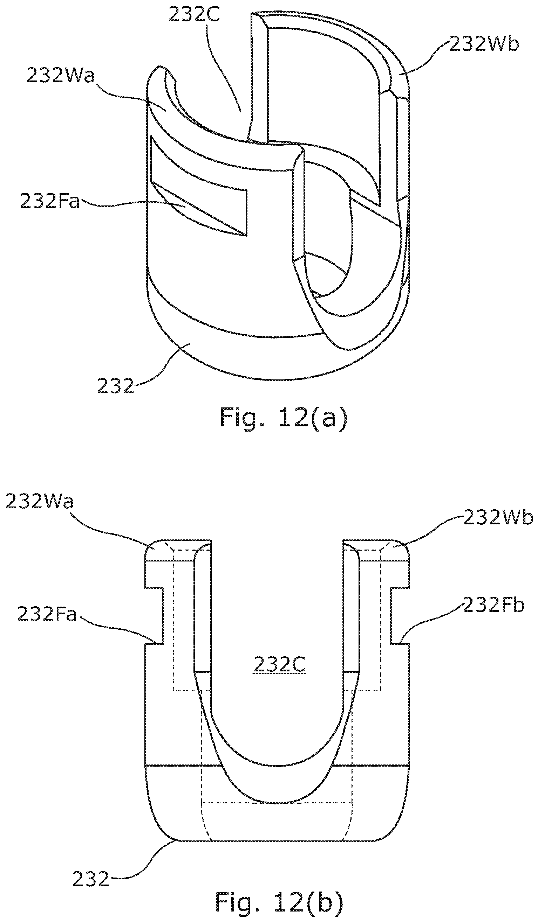

The anchoring device 140 includes a pair of oppositely arranged flanged or seating or bearing portions 142a, 142b, each of which is integral with the first, main body portion of the device 140 defining the central hole/aperture 148. The inner perimeter of the central hole/aperture 148 is sized and shaped to substantially match the outer periphery of the head portion (132 in the case of the pedicle screw of FIG. 9, or 232 in the case of the pedicle screw of FIG. 12) of the relevant pedicle screw over which the device 140 is to be fitted. The first flanged or seating or bearing portion 142a is configured so as to be bent or curved or ramped upwardly relative to the first, main body portion, with the second flanged or seating or bearing portion 142b optionally also being slightly (e.g. to a lesser extent) curved or bent or ramped in either the same or the opposite direction relative to the first, main body portion of the device 140.

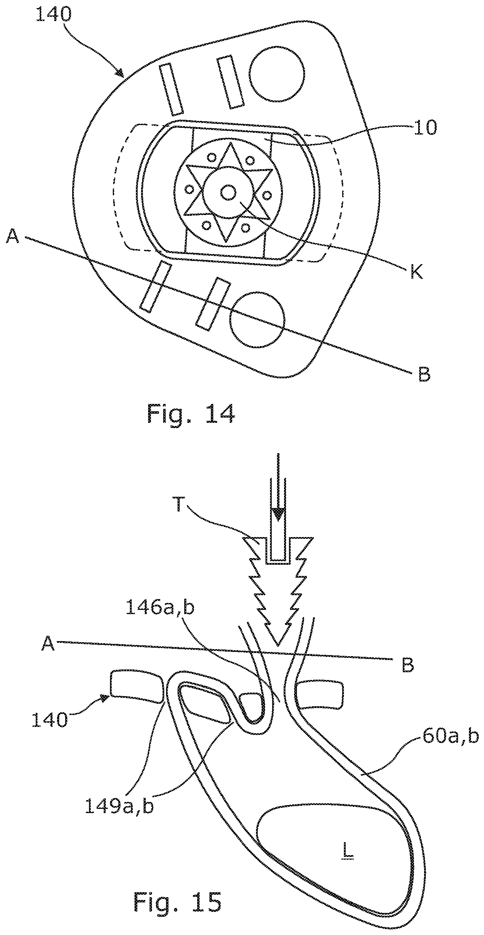

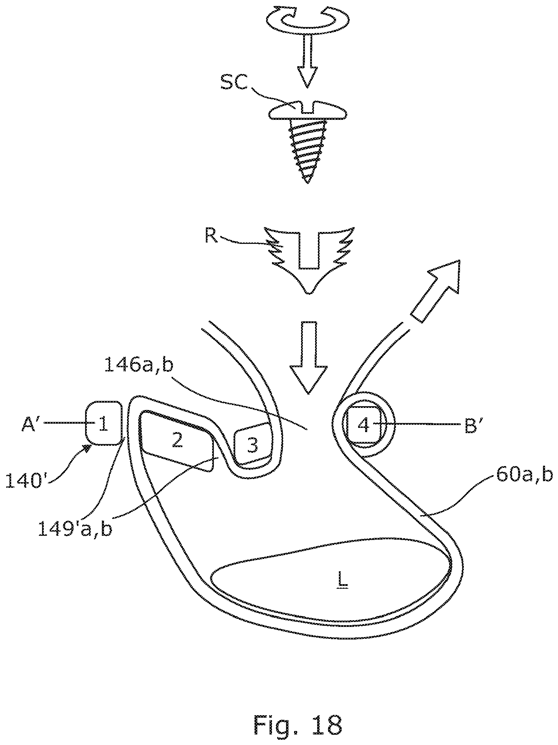

For the purpose of anchoring and securing to the device 140 the relevant sub-laminar band(s) or tape(s) 60a, 60b, the construction and configuration of the second portions of the device are also simplified. As shown in FIGS. 10 and 11, instead of the distinct hoop- or loop-like second portions 46a, 46b together with their respective slot-containing inserts 50a, 50b mounted therein forming the means by which the relevant sub-laminar band(s) or tape(s) 60a, 60b are anchored and secured to the device as in the first embodiments described above, in this mechanically simpler arrangement of this embodiment each respective opposed second portion of the anchoring device 140, to either side of the central hole/aperture 148, is formed with a circular or rounded hole or aperture 146a, 146b extending through the material thereof, and adjacent thereto is further provided a pair of side-by-side but slightly spaced apart (e.g. a few, such as from about 2 or 3 to about 10 mm) and approx. parallel elongated slots or slits 149a, 149b (also extending through the material thereof) for having the relevant respective ones of the sub-laminar bands or tapes 60a, 60b passed therethrough, tensioned or tightened as necessary, and finally secured in place relative thereto. Such final securing may be effected or aided by means of a suitable respective locking screw, plug or like insertable locking or clamping element (e.g. T or SC, as shown in FIGS. 15, 17 & 18), optionally in combination with a suitable grommet, collar or similar insert (e.g. R in FIG. 18), which locking or clamping element T, SC is most usefully insertable into the relevant circular/rounded hole or aperture 146a, 146b.

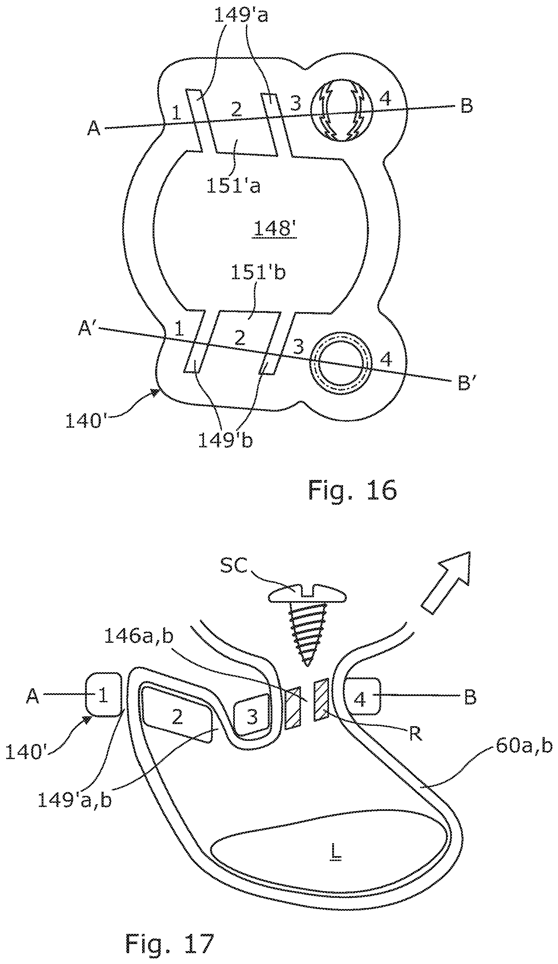

As shown in the embodiment device 140 of FIGS. 10 & 11, the defining perimeters of each of the circular/rounded holes/apertures 146a, 146b and also the defining perimeters of the various anchoring/securing slots or slits 149a, 149b are all wholly contained within the material of the device 140 in which they are formed, as also seen in FIG. 14. However, in certain other alternative embodiment forms of device 140' such as that shown in FIG. 16, the defining perimeters of the various anchoring/securing slots or slits 149'a, 149'b are only partially contained within the material of the device 140' in which they are formed, so that each respective slot/slit 149'a, 149'b is open and unbounded on one short side, i.e. a radially inner side, thereof. This alternative configuration of such slots/slits 149'a, 149'b as in FIG. 16 serves to create a respective tab or tongue 151'a, 151'b between each respective pair thereof. Each tab or tongue 151'a, 151'b may facilitate the overall insertion and threading through the slots/slits 149'a, 149'b of the respective sub-laminar bands or tapes 60a, 60b and/or may also serve as a deformable or displaceable member that forms its own interference fit against the outer sidewall of the pedicle screw head portion once it has been inserted in to the central hole/aperture of the device 140'.

For use in combination with the alternative embodiment form of anchoring device 140 (or 140') as shown in FIGS. 10 & 11 (and also FIG. 16), a pedicle screw may be employed of various designs - which may be either of a known design (as described below in relation to FIG. 12, for example) or alternatively may be a novel alternatively modified pedicle screw 130 as shown in FIGS. 9(a) and 9(b). In that alternatively modified pedicle screw 130 its head portion 132 still includes "second interengagement means" formed on or in its radially outer sidewall, but now those "second interengagement means" comprise a circumferential, or alternatively at least one pair of oppositely or diametrically oppositely arranged, externally extending flange(s), lip(s), step(s) or land(s) 132Fa, 132Fb formed in the head portion 132's outer sidewall. Thus, the respective flanged or seating or bearing portions 142a, 142b of the anchoring device 140 can engage with the respective external flange(s), lip(s), step(s) or land(s) 132Fa, 132Fb by virtue of a simple mechanical abutment or seating or bearing relationship.

The remaining structural features of the alternatively modified pedicle screw 130 of FIGS. 9(a) & 9(b) that are identified by reference numerals not yet referred to as such correspond in form and function to those respective features of the first described modified pedicle screw 30 of FIGS. 6(a) & 6(b) which are referred to by the same basic reference numeral but in the context of the embodiment of FIGS. 9(a) & 9(b) are incremented by 100. Thus, these other labelled structural features in FIGS. 9(a) & 9(b) do not need describing again here.

However, the alternative embodiment form of anchoring device 140 of FIGS. 10 & 11 (and also FIG. 16) can also be used in combination with certain known designs of pedicle screw, such as that labelled as 230 whose head portion 232 is shown in FIGS. 12(a) and 12(b). Again, illustrated features of this known pedicle screw head portion 232 which corresponding in form and function to respective features of the first described modified pedicle screw 30 of FIGS. 6(a) & 6(b) are labelled with the same basic reference numeral but incremented by another 100. In this design of pedicle screw head portion 232, the outer sidewalls 232Wa, 232Wb of the body portion of the head portion 232 include a pair of externally facing, diametrically opposed, recesses or recessed lands or indentations or shoulders 232Fa, 232Fb, each of which presents a flat seating or bearing surface on its lower side against which can abut or sit the respective flanged or seating or bearing portions 142a, 142b of the anchoring device 140.

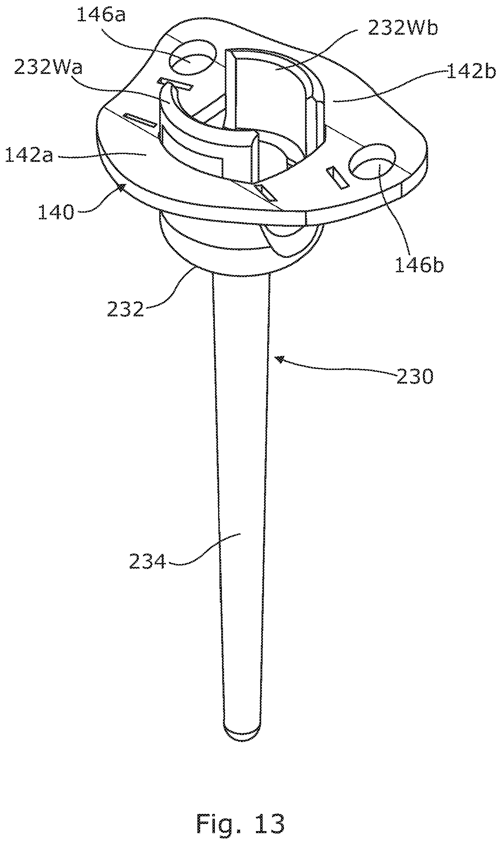

FIG. 13 shows the combination of the alternative embodiment anchoring device 140 of FIGS. 10 & 11 together with the known pedicle screw 230 of FIG. 12, showing them in their attached and united condition. As can be appreciated from FIG. 13, the respective flanged or seating or bearing portions 142a, 142b of the anchoring device 140 and the respective recesses or recessed lands or indentations or shoulders 232Fa, 232Fb of the pedicle screw head portion 232 of the screw 230 (or alternatively the external flanges, lips, steps or lands 132Fa, 132Fb of the pedicle screw head portion 132 of the screw 130 of FIG. 9, as the case may be) are overall shaped, dimensioned and positioned relative to each other such that they substantially match or approximate in configuration and fit together with one another in respective pairs thereof such that they can readily unite with each other in a simple abutment or seating or bearing manner, and thus so that the anchoring device 140 and the pedicle screw head portion 232 (or 132) can be readily unitable by virtue of a simple mechanical slide-over or push-fit or friction-fit connection. Moreover, a further advantage of the combined arrangement as shown in FIG. 13 is that the stability of the anchoring device 140 on the screw 230 may come predominantly (or even substantially solely) from the existing geometry of the screw head portion 232. This may thus facilitate use of such an alternative design of anchoring device 140 with existing designs of pedicle screws (e.g. 230), which can lead to lower overall manufacturing costs of new and improved anchoring arrangements provided by embodiments of the present invention.

FIG. 14 shows the attached/united combination of FIG. 13, together with a locking screw K anchoring a stabilising rod 10 extending through the pedicle screw head portion 232, showing the combination ready for receiving various sub-laminar bands or tapes 60a, 60b passing through the various apertures and/or slots/slits thereof.

By way of another practical example only, this time directed specifically to the use of the alternative anchoring device 140 as described in detail above, a summary of the sequence of principal steps used in the deployment of this particular alternative embodiment anchoring arrangement described above may be as follows:
1. The pedicle screw hole is prepared to receive a screw in a standard fashion.
2. The modified screw 130 (or alternatively the known screw 230) is inserted in the usual fashion.
3. The sub-laminar bands 60a, 60b are passed beneath the laminae at the chosen levels.
4. The ends of the bands 60a, 60b are threaded into the anchoring device 140, via the holes and slots/slits 146a, 149a; 146b, 149b, and redundant material is slowly pulled through.
5. As the band tensioning occurs, the device 140 is seated onto the screw head 132 (or 232).
6. Final tensioning of the bands 60a, 60b occurs simultaneously and the tension is maintained by inserting an interference-fit locking/clamping insert T, SC, R etc in the relevant hole/aperture 146a, 146b.
7. The excess band material (if any) is then excised with a blade, leaving e.g. around 5 mm of excess band only.
8. Manipulation of the spine through the screw 130 (or 230) and reduction of the screw 130 (or 230) to the rod 10 may then occur.
9. Once the rod 10 is seated, a locking screw K is secured to the rod 10.
10. Final manipulation and derotation may be performed, as necessary.
11. Final tightening of the locking screw K may occur.

FIG. 15 is a simplified schematic drawing showing one example way in which a sub-laminar band or tape 60a or 60b can be passed through the various apertures/slots/slits of the anchoring device 140 and secured in place therein in order to anchor the band or tape 60a or 60b with respect to a spinal vertebral lamina L during corrective spinal vertebral surgery. The line A → B represents the direction of passage of the band or tape 60a,b as it is threaded through the various apertures/slots/slits of the device 140.

FIG. 16 shows the slightly modified embodiment of anchoring device 140' with a slightly different configuration of band/tape anchoring/securement slots 149'a, 149'b, as has already been described above.

In order to further explain some of the possible methods for securing the tensioned sub-laminar bands/tapes 60a, 60b to the anchoring device 140' (or even 140), the following two options present themselves, as will now be described with reference to FIGS. 16, 17 and 18:
Once a band/tape 60a,b has been passed beneath the lamina bone L, it needs to be secured to the anchoring device 140', put under appropriate tension and then locked in place with that tension. These steps may be carried out as follows:
(i) Band passage:
   (a) Option 1 (referring to FIGS. 16 & 17 - and following the direction of line A → B):
      - The band is looped through the various apertures/slots/slits as per FIG. 17.
   (b) Option 2 (referring to FIGS. 16 & 18 - and following the direction of line A' → B'):
      - One limb of the band/tape 60a,b is looped through the various apertures/slots/slits in a similar way to Option 1 moving from A towards B. In this Option 2, however, the last section of the anchoring device 140' (annotated "4" in these FIGS.) is machined to be circular in cross-section (FIG. 18). The limb of the band/tape 60a,b in this situation is passed twice through the large hole/aperture 146'a,b.
(ii) Band tensioning:
   (a) Option 1 (referring to FIGS. 16 & 17 - and following the direction of line A → B:
      - Once the band/tape 60a,b is fully passed, it is tensioned manually and then with a tensioning device, to maintain the desired tension. A screw SC is inserted into the largest of the holes/apertures 146'a,b which causes a crimping of the band/tape material in order to securely hold the band/tape 60a,b in its tensioned position (FIG. 17).
   (b) Option 2 (referring to FGS. 16 & 18 - and following the direction of line A' → B':
      - Because of the smooth circular finish to section "4" of the device 140', the limb of the band/tape 60a,b is still amenable to manual tensioning, despite it being looped around the device 140'. Once manual tensioning is achieved, final tensioning occurs with a tensioning device which holds the desired tension. A Rawlplug-like screw insert or anchor R is inserted into the largest hole/aperture 146'a,b from above, and a ~2 mm screw SC inserted to definitively secure the tension in the band/tape 60a,b.
(iii) Once tensioning is secured, the bands/tapes are cut ~5 mm from the securing interfaces.

It is to be understood that the above description of some specific embodiments of the invention in terms of their various features and aspects has been by way of non-limiting example(s) only, and various modifications may be made from what has been specifically described and illustrated whilst remaining within the scope of the invention as set out in the appended claims.

Throughout the description and claims of this specification, the words "comprise" and "contain" and linguistic variations of those words, for example "comprising" and "comprises", mean "including but not limited to", and are not intended to (and do not) exclude other moieties, additives, components, elements, integers or steps.

Throughout the description and claims of this specification, the singular encompasses the plural unless expressly stated otherwise or the context dictates otherwise. In particular, where the indefinite article is used, the specification is to be understood as contemplating plurality as well as singularity, unless expressly stated otherwise or the context dictates requires otherwise.

Furthermore, through this specification any feature, component, element, integer, characteristic, property, compound, chemical moiety or group described in conjunction with a particular aspect, embodiment or example of the invention is/are to be understood to be applicable to any other aspect, embodiment or example described herein unless incompatible therewith or expressly stated otherwise.

## Claims

1. An anchoring device (40) for anchoring one or more sub-laminar bands or tapes (60a, 60b) to a pedicle screw (30), the device (40) comprising:
a first portion (41) configured for attachment to a head portion (32) of the pedicle screw (30) by interengagement with said head portion (32); and
a plurality of second portions (46a, 46b) for anchoring thereto one or more sub-laminar bands or tapes (60a, 60b);
wherein the first portion (41) includes first interengagement means (42Na, 42Nb) for interengagement with the head portion (32) of the pedicle screw (30) to effect the said interengagement attachment of the first portion (41) of the anchoring device thereto;
wherein the said plurality of second portions (46a, 46b) are arranged equi-angularly or symmetrically around the first portion (41) of the device (40), each second portion (46a, 46b) being configured and arranged for anchoring thereto a said sub-laminar band or tape (60a, 60b), and each said second portion (46a, 46b) including, or being configured as, an apertured or hollow hoop- or loop-like portion or extension located on or extending from diametrically opposite sides of the first portion (41) of the device and extending in radially opposite directions therefrom,
and further wherein each of the second portions (46a, 46b) has a general plane in which it lies and the second portions (46a, 46b) are configured such that the respective general planes of the second portions (46a, 46b) are substantially co-planar with each other.

2. An anchoring device (40) according to claim 1, wherein the first portion (41) is configured for attachment to the head portion (32) of the pedicle screw (30) by interengagement of the first interengagement means (42Na, 42Nb) with at least a radially outer sidewall portion of the screw head portion (32);
optionally wherein the first portion (41) is configured such that, upon interengagement of the first interengagement means (42Na, 42Nb) with the outer sidewall portion of the screw head portion (32), at least a lower part of the first portion (41) of the device (40) adjacent the first interengagement means (42Na, 42Nb) abuts or lies adjacent or engages the lower sidewall portion of the screw head portion (32).

3. An anchoring device (40) according to claim 1 or claim 2, wherein the first portion (41) of the device is configured such that it is attachable to the head portion (32) of the pedicle screw (30), via the interengagement therewith of the first interengagement means (42Na, 42Nb), by means of insertion of a screw-threaded shaft (34) of the pedicle screw (30), which shaft (34) extends from a base (32L) of the head portion (32), through an aperture (48) in the first portion (41) of the device in a direction away from the first portion (41) of the device and relative movement towards one another of the first portion (41) of the device and the base (32L) of the screw head portion (32).

4. An anchoring device (40) according to any preceding claim, wherein the first portion (41) comprises an apertured or hollow base portion and one or more, optionally at least one pair of mutually oppositely arranged, attachment portions (42a, 42b) extending generally perpendicularly therefrom;
optionally wherein either one or both of the following (i) and (ii) is/are satisfied:
(i) wherein the attachment portions (42a, 42b) are configured as a pair of diametrically oppositely arranged protruding lugs, tabs or arms (42a, 42b) extending perpendicularly from the base portion on diametrically opposite sides thereof, and are disposed so as to be generally substantially parallel to one other and configured to interengage with the outer sidewall of the head portion (32) of the screw (30) on diametrically opposite sides of the screw head portion (32);
(ii) wherein the attachment portions (42a, 42b) each terminate at a distal end thereof (that being the end thereof opposite a proximal end thereof at which the respective attachment portion (42a, 42b) is joined to the base portion of the first portion (41) of the device) in a radially inwardly projecting interengagement nose portion (42Na, 42Nb), whereby the interengagement nose portions (42Na, 42Nb) of the pair of attachment portions (42a, 42b) point generally towards one another from opposite sides, especially diametrically opposite sides, of the first portion (41) of the device.

5. An anchoring device (40) according to claim 4, wherein (ii) is satisfied, and wherein each interengagement nose portion (42Na, 42Nb) includes an obliquely inclined interengagement surface (44Fa, 44Fb) therebeneath, wherein either:
(a) the angle of inclination of each respective interengagement surface (44Fa, 44Fb) is such that a radially inner end of each respective nose portion (42Na, 42Nb) is closer to the base portion of the first portion (41) of the device than is a radially outer end of the respective nose portion (42Na, 42Nb); or
(b) each interengagement nose portion (42Na, 42Nb) comprises a said interengagement surface (44Fa, 44Fb), which is that surface directed towards the base portion of the first portion (41) of the device, which forms an angle of less than 90 degrees, optionally an angle in the range of from 50 or 60 or 70 to 80 or 85 or 87 degrees, with a radially inner side surface of the respective attachment portion (42a, 42b) to which the respective nose portion (42Na, 42Nb) is joined.

6. An anchoring device (40) according to either claim 4, wherein (ii) is satisfied, or claim 5, and wherein the respective interengagement nose portions (42Na, 42Nb) of the pair of attachment portions (42a, 42b) form a snap-fit attachment mechanism with second interengagement means (32Na, 32Nb) on the head portion (32) of the pedicle screw (30).

7. An anchoring device (40) according to any preceding claim, further wherein the second portions (46a, 46b) are configured such that the respective general planes of the second portions (46a, 46b) are substantially co-planar with a general plane of a base portion of the anchoring device (40) from which extend the first interengagement means (42Na, 42Nb) which provide the interengagement attachment of the first portion (41) of the device to the pedicle screw head portion (32).

8. An anchoring device (40) according to any preceding claim, wherein each of the second portions (46a, 46b) has mounted therein an insert (50a, 50b) for providing the means of anchoring to that second portion (46a, 46b) of the device the or the respective sub-laminar band or tape (60a, 60b);
and wherein the or each insert (50a, 50b) comprises a central body (51a, 51b), in which there is provided a slot or aperture (49a, 49b) through which is able to pass or be accommodated the or the respective sub-laminar band or tape (60a, 60b), and an outer frame configured for mounting the insert (50a, 50b) in the respective second portion (46a, 46b) of the device,
optionally wherein the or each outer frame is configured to be engageable in the respective second portion (46a, 46b) of the device by means of a respective snap-fit connection.

9. An anchoring device (40) according to claim 8, wherein either one or both of the following (i) and (ii) is/are satisfied:
(i) wherein the central body (51a, 51b) of the or the respective insert (50a, 50b) takes the form of a short circular cylinder, which is mounted freely rotatably within a geometrically similar circular mounting within the respective frame, so that the orientation of the slot or aperture (49a, 49b) therein may vary or be self-adjusting so as to adopt an optimum orientation as dictated by the various forces that may be exerted on the anchoring device (40) by the one or more sub-laminar bands or tapes (60a, 60b) once they have been anchored to the device and tightened into their desired secured or locked condition;
(ii) wherein either:
(a) the slot or aperture (49a, 49b) within the respective central body (51a, 51b) of each respective insert (50a, 50b) is dimensioned to allow the respective sub-laminar band or tape (60a, 60b) to pass and slide freely therethrough, and a separate discrete securement or locking device is provided for securing the respective sub-laminar band or tape (60a, 60b) in its tightened condition once it has been deployed in its desired configuration having been passed or threaded around the relevant vertebra(e) and anchored to the anchoring device (40); or
(b) the or each respective slot or aperture (49a, 49b) within the respective central body (51a, 51b) of the respective insert (50a, 50b) itself includes a securement or locking device which allows the respective sub-laminar band or tape (60a, 60b) to be secured directly within the respective insert (50a, 50b) of the device.

10. An anchoring device (140) according to claim 1 or claim 2, or according to claim 4 or claim 7 when dependent on claim 1 or claim 2, wherein the first portion of the device (140) is configured such that it is attachable to the head portion (132) of the pedicle screw (130), via the interengagement therewith of the first interengagement means (142a, 142b), by means of placement of a hole or aperture (148) in the first portion of the device (140) over the pedicle screw's head portion (132) from thereabove, i.e. from the side thereof opposite to that from which extends the pedicle screw's threaded shaft (134), and the anchoring device (140) and the head portion (132) of the pedicle screw (130) are attachable and unitable by virtue of a push-fit or friction-fit or abutment-fit connection.

11. An anchoring device (140) according to claim 10, wherein any one or more of the following (i), (ii) and (iii) is/are satisfied:
(i) wherein the anchoring device (140) is formed as a substantially unitary one-piece structure,
optionally wherein the anchoring device (140) is configured with at least one portion (142a) thereof curved or bent or concave or convex or ramped or stepped with respect to the remainder of the device (140);
(ii) wherein the first interengagement means (142a, 142b) comprises at least one pair of oppositely or diametrically oppositely arranged, flanged or seating or bearing portions (142a, 142b) of the device;
(iii) wherein each second portion of the device (140) comprises one or more, or a plurality of, holes or apertures or slots or slits (146a, 146b; 149a, 149b) formed in and/or extending through the material of the respective second portion.

12. An anchoring device (140) according to claim 11, wherein (iii) is satisfied, and wherein:
either (a) the defining perimeter(s) of any one or more of the said hole(s)/aperture(s) or slots/slits (146a, 146b) is/are wholly contained within the material of the relevant second portion of the device in which it/they is/are formed;
or (b) the defining perimeter(s) of any two of the said hole(s)/aperture(s) or slots/slits (149a, 149b) is/are only partially contained within the material of the relevant second portion of the device (140) in which they are formed, so that the relevant hole(s)/aperture(s) or slots/slits (149a, 149b) is/are open and unbounded on at least one side or portion thereof, thereby creating a or a respective tab or tongue (151'a, 151'b) inbetween at least one pair thereof.

13. An anchoring arrangement for anchoring one or more sub-laminar bands or tapes (60a, 60b) with respect to one or more spinal vertebrae in corrective spinal vertebral surgery, the arrangement comprising:
a pedicle screw (30; 130) for insertion into a selected vertebral pedicle; and
an anchoring device (40; 140) according to any one of claims 1 to 12;
optionally wherein the head portion (32; 132) of the pedicle screw (30; 130) comprises second interengagement means (32Na, 32Nb; 132Fa, 132Fb) configured for interengagement with the first interengagement means (42Na, 42Nb; 142a, 142b) provided on the first portion (41; 141) of the anchoring device (40, 140), whereby the first and second interengagement means (42Na, 42Nb; 142a, 142b; 32Na, 32Nb; 132Fa, 132Fb) are each configured so that they together, once they are interengaged with each other, effect the said interengagement attachment of the first portion (41; 141) of the anchoring device (40; 140) to the head portion (32; 132) of the pedicle screw (30; 130).

14. A kit or set of component parts for use in corrective spinal vertebral surgery, the kit or set comprising:
at least one pedicle screw (30; 130) for insertion into a selected vertebral pedicle of one or more spinal vertebrae; and
at least one anchoring device (40; 140) according to any one of claims 1 to 12;
optionally together with one or more sub-laminar bands or tapes (60a, 60b) for anchoring with respect to said one or more spinal vertebrae in said corrective spinal vertebral surgery.

## Patentansprüche

1. Verankerungsvorrichtung (40) zum Verankern eines oder mehrerer sublaminärer Bänder oder Streifen (60a, 60b) an einer Pedikelschraube (30), die Vorrichtung (40) umfassend:
einen ersten Abschnitt (41), der für eine Befestigung an einem Kopfabschnitt (32) der Pedikelschraube (30) durch Eingriff mit dem Kopfabschnitt (32) konfiguriert ist; und
eine Vielzahl von zweiten Abschnitten (46a, 46b), um daran ein oder mehrere sublaminäre Bänder oder Streifen (60a, 60b) zu verankern;
wobei der erste Abschnitt (41) erste Eingriffsmittel (42Na, 42Nb) für einen Eingriff mit dem Kopfabschnitt (32) der Pedikelschraube (30) einschließt, um die Eingriffsbefestigung des ersten Abschnitts (41) der Verankerungsvorrichtung daran zu bewirken;
wobei die Vielzahl von zweiten Abschnitten (46a, 46b) gleichwinklig oder symmetrisch um den ersten Abschnitt (41) der Vorrichtung (40) herum angeordnet ist, wobei jeder zweite Abschnitt (46a, 46b) konfiguriert und angeordnet ist, um daran ein sublaminäres Band oder einen Streifen (60a, 60b) zu verankern, und jeder zweite Abschnitt (46a, 46b) einen mit Öffnungen versehenen oder hohlen reifen- oder schlaufenartigen Abschnitt oder eine derartige Verlängerung einschließt oder als ein solcher konfiguriert ist, der/die an diametral gegenüberliegenden Seiten des ersten Abschnitts (41) der Vorrichtung angeordnet ist oder sich davon erstreckt und sich in radial entgegengesetzte Richtungen davon erstreckt,
und wobei ferner jeder der zweiten Abschnitte (46a, 46b) eine Hauptebene aufweist, in der er liegt, und die zweiten Abschnitte (46a, 46b) so konfiguriert sind, dass die jeweiligen Hauptebenen der zweiten Abschnitte (46a, 46b) im Wesentlichen koplanar zueinander sind.

2. Verankerungsvorrichtung (40) nach Anspruch 1, wobei der erste Abschnitt (41) für eine Befestigung an dem Kopfabschnitt (32) der Pedikelschraube (30) durch Eingriff der ersten Eingriffsmittel (42Na, 42Nb) mit zumindest einem radial äußeren Seitenwandabschnitt des Schraubenkopfabschnitts (32) konfiguriert ist;
wobei optional der erste Abschnitt (41) so konfiguriert ist, dass beim Eingriff der ersten Eingriffsmittel (42Na, 42Nb) mit dem äußeren Seitenwandabschnitt des Schraubenkopfabschnitts (32) zumindest ein unterer Teil des ersten Abschnitts (41) der Vorrichtung (40) benachbart zu den ersten Eingriffsmitteln (42Na, 42Nb) an dem unteren Seitenwandabschnitt des Schraubenkopfabschnitts (32) anliegt oder benachbart dazu liegt oder damit in Eingriff steht.

3. Verankerungsvorrichtung (40) nach Anspruch 1 oder Anspruch 2, wobei der erste Abschnitt (41) der Vorrichtung so konfiguriert ist, dass er an dem Kopfabschnitt (32) der Pedikelschraube (30), über den Eingriff der ersten Eingriffsmittel (42Na, 42Nb) damit, durch Einführen eines mit Schraubengewinde versehenen Schafts (34) der Pedikelschraube (30), wobei der Schaft (34) sich von einer Basis (32L) des Kopfabschnitts (32) erstreckt, durch eine Öffnung (48) in dem ersten Abschnitt (41) der Vorrichtung in einer Richtung weg von dem ersten Abschnitt (41) der Vorrichtung und durch eine relative Bewegung des ersten Abschnitts (41) der Vorrichtung und der Basis (32L) des Schraubenkopfabschnitts (32) aufeinander zu befestigbar ist.

4. Verankerungsvorrichtung (40) nach einem der vorstehenden Ansprüche, wobei der erste Abschnitt (41) einen mit Öffnungen versehenen oder hohlen Basisabschnitt und einen oder mehrere, optional mindestens ein Paar von einander gegenüberliegend angeordneten, Befestigungsabschnitte (42a, 42b) umfasst, die sich im Allgemeinen senkrecht davon erstrecken;
wobei optional entweder eine oder beide der folgenden (i) und (ii) erfüllt ist/sind:
(i) wobei die Befestigungsabschnitte (42a, 42b) als ein Paar von diametral gegenüberliegend angeordneten vorstehenden Laschen, Zungen oder Armen (42a, 42b) konfiguriert sind, die sich senkrecht von dem Basisabschnitt an diametral gegenüberliegenden Seiten davon erstrecken, und so angeordnet sind, dass sie im Allgemeinen im Wesentlichen parallel zueinander sind und so konfiguriert sind, dass sie mit der äußeren Seitenwand des Kopfabschnitts (32) der Schraube (30) an diametral gegenüberliegenden Seiten des Schraubenkopfabschnitts (32) in Eingriff kommen;
(ii) wobei die Befestigungsabschnitte (42a, 42b) jeweils an einem distalen Ende davon (das das Ende davon ist, das einem proximalen Ende davon gegenüberliegt, an dem der jeweilige Befestigungsabschnitt (42a, 42b) mit dem Basisabschnitt des ersten Abschnitts (41) der Vorrichtung verbunden ist) in einem radial nach innen vorstehenden Eingriffsnasenabschnitt (42Na, 42Nb) enden, wodurch die Eingriffsnasenabschnitte (42Na, 42Nb) des Paares von Befestigungsabschnitten (42a, 42b) im Allgemeinen von gegenüberliegenden Seiten, insbesondere diametral gegenüberliegenden Seiten, des ersten Abschnitts (41) der Vorrichtung aufeinander zu weisen.

5. Verankerungsvorrichtung (40) nach Anspruch 4, wobei (ii) erfüllt ist, und wobei jeder Eingriffsnasenabschnitt (42Na, 42Nb) eine schräg geneigte Eingriffsfläche (44Fa, 44Fb) darunter einschließt, wobei entweder:
(a) der Neigungswinkel jeder jeweiligen Eingriffsfläche (44Fa, 44Fb) so ist, dass ein radial inneres Ende jedes jeweiligen Nasenabschnitts (42Na, 42Nb) näher an dem Basisabschnitt des ersten Abschnitts (41) der Vorrichtung liegt als ein radial äußeres Ende des jeweiligen Nasenabschnitts (42Na, 42Nb); oder
(b) jeder Eingriffsnasenabschnitt (42Na, 42Nb) eine Eingriffsfläche (44Fa, 44Fb) umfasst, die diejenige Fläche ist, die in Richtung des Basisabschnitts des ersten Abschnitts (41) der Vorrichtung gerichtet ist, die einen Winkel von weniger als 90 Grad, optional einen Winkel im Bereich von 50 oder 60 oder 70 bis 80 oder 85 oder 87 Grad, mit einer radial inneren Seitenfläche des jeweiligen Befestigungsabschnitts (42a, 42b) bildet, mit dem der jeweilige Nasenabschnitt (42Na, 42Nb) verbunden ist.

6. Verankerungsvorrichtung (40) nach entweder Anspruch 4, wobei (ii) erfüllt ist, oder Anspruch 5, und wobei die jeweiligen Eingriffsnasenabschnitte (42Na, 42Nb) des Paares von Befestigungsabschnitten (42a, 42b) einen Schnappbefestigungsmechanismus mit zweiten Eingriffsmitteln (32Na, 32Nb) an dem Kopfabschnitt (32) der Pedikelschraube (30) bilden.

7. Verankerungsvorrichtung (40) nach einem der vorstehenden Ansprüche, wobei ferner die zweiten Abschnitte (46a, 46b) so konfiguriert sind, dass die jeweiligen Hauptebenen der zweiten Abschnitte (46a, 46b) im Wesentlichen koplanar zu einer Hauptebene eines Basisabschnitts der Verankerungsvorrichtung (40) sind, von der sich die ersten Eingriffsmittel (42Na, 42Nb) erstrecken, die die Eingriffsbefestigung des ersten Abschnitts (41) der Vorrichtung an dem Pedikelschraubenkopfabschnitt (32) bereitstellen.

8. Verankerungsvorrichtung (40) nach einem der vorstehenden Ansprüche, wobei in jedem der zweiten Abschnitte (46a, 46b) ein Einsatz (50a, 50b) montiert ist, um die Mittel zum Verankern des oder des jeweiligen sublaminären Bandes oder Streifens (60a, 60b) an diesem zweiten Abschnitt (46a, 46b) der Vorrichtung bereitzustellen;
und wobei der oder jeder Einsatz (50a, 50b) einen zentralen Körper (51a, 51b) umfasst, in dem ein Schlitz oder eine Öffnung (49a, 49b) bereitgestellt ist, durch den bzw. die das oder der jeweilige sublaminäre Band oder Streifen (60a, 60b) hindurchtreten kann oder aufgenommen werden kann, und einen äußeren Rahmen, der für die Montage des Einsatzes (50a, 50b) in dem jeweiligen zweiten Abschnitt (46a, 46b) der Vorrichtung konfiguriert ist,
wobei optional der oder jeder äußere Rahmen so konfiguriert ist, dass er in dem jeweiligen zweiten Abschnitt (46a, 46b) der Vorrichtung mittels einer jeweiligen Schnappverbindung in Eingriff bringbar ist.

9. Verankerungsvorrichtung (40) nach Anspruch 8, wobei entweder eine oder beide der folgenden (i) und (ii) erfüllt ist/sind:
(i) wobei der zentrale Körper (51a, 51b) des oder des jeweiligen Einsatzes (50a, 50b) die Form eines kurzen Kreiszylinders annimmt, der frei drehbar innerhalb einer geometrisch ähnlichen kreisförmigen Halterung innerhalb des jeweiligen Rahmens montiert ist, sodass die Ausrichtung des Schlitzes oder der Öffnung (49a, 49b) darin variieren kann oder selbsteinstellend ist, um eine optimale Ausrichtung anzunehmen, wie sie durch die verschiedenen Kräfte vorgeschrieben wird, die auf die Verankerungsvorrichtung (40) durch das eine oder die mehreren sublaminären Bänder oder Streifen (60a, 60b) ausgeübt werden können, sobald sie an der Vorrichtung verankert und in ihren gewünschten gesicherten oder verriegelten Zustand festgezogen wurden;
(ii) wobei entweder:
(a) der Schlitz oder die Öffnung (49a, 49b) innerhalb des jeweiligen zentralen Körpers (51a, 51b) jedes jeweiligen Einsatzes (50a, 50b) so dimensioniert ist, dass das jeweilige sublaminäre Band oder der Streifen (60a, 60b) frei dort hindurchtreten und gleiten kann, und eine separate, diskrete Befestigungs- oder Verriegelungsvorrichtung bereitgestellt ist, um das jeweilige sublaminäre Band oder den Streifen (60a, 60b) in seinem festgezogenen Zustand zu sichern, sobald es in seiner gewünschten Konfiguration eingesetzt wurde, nachdem es um den relevanten Wirbel bzw. die relevanten Wirbel herumgeführt oder gefädelt und an der Verankerungsvorrichtung (40) verankert wurde; oder
(b) der oder jeder jeweilige Schlitz oder die Öffnung (49a, 49b) innerhalb des jeweiligen zentralen Körpers (51a, 51b) des jeweiligen Einsatzes (50a, 50b) selbst eine Befestigungs- oder Verriegelungsvorrichtung einschließt, die es ermöglicht, das jeweilige sublaminäre Band oder den Streifen (60a, 60b) direkt innerhalb des jeweiligen Einsatzes (50a, 50b) der Vorrichtung zu sichern.

10. Verankerungsvorrichtung (140) nach Anspruch 1 oder Anspruch 2 oder nach Anspruch 4 oder Anspruch 7, wenn abhängig von Anspruch 1 oder Anspruch 2, wobei der erste Abschnitt der Vorrichtung (140) so konfiguriert ist, dass er an dem Kopfabschnitt (132) der Pedikelschraube (130) über den Eingriff der ersten Eingriffsmittel (142a, 142b) damit durch Platzieren eines Lochs oder einer Öffnung (148) in dem ersten Abschnitt der Vorrichtung (140) über dem Kopfabschnitt (132) der Pedikelschraube von oben, d. h. von der Seite davon, die derjenigen gegenüberliegt, von der sich der Gewindeschaft (134) der Pedikelschraube erstreckt, befestigbar ist, und die Verankerungsvorrichtung (140) und der Kopfabschnitt (132) der Pedikelschraube (130) mittels einer Steck-, Reibschluss- oder Anlageverbindung befestigbar und vereinigbar sind.

11. Verankerungsvorrichtung (140) nach Anspruch 10, wobei ein beliebiges oder mehrere der folgenden (i), (ii) und (iii) erfüllt ist/sind:
(i) wobei die Verankerungsvorrichtung (140) als eine im Wesentlichen einheitliche einteilige Struktur ausgebildet ist,
wobei optional die Verankerungsvorrichtung (140) so konfiguriert ist, dass mindestens ein Abschnitt (142a) davon in Bezug auf den Rest der Vorrichtung (140) gekrümmt oder gebogen oder konkav oder konvex oder rampenförmig oder abgestuft ist;
(ii) wobei die ersten Eingriffsmittel (142a, 142b) mindestens ein Paar von gegenüberliegend oder diametral gegenüberliegend angeordneten geflanschten oder aufliegenden oder tragenden Abschnitten (142a, 142b) der Vorrichtung umfassen;
(iii) wobei jeder zweite Abschnitt der Vorrichtung (140) ein oder mehrere oder eine Vielzahl von Löchern oder Öffnungen oder Schlitzen oder Spalten (146a, 146b; 149a, 149b) umfasst, die in dem Material des jeweiligen zweiten Abschnitts ausgebildet sind und/oder sich durch dieses erstrecken.

12. Verankerungsvorrichtung (140) nach Anspruch 11, wobei (iii) erfüllt ist, und wobei:
entweder (a) der begrenzende Umfang (die begrenzenden Umfänge) eines beliebigen oder mehrerer der Löcher/Öffnungen oder Schlitze/Spalten (146a, 146b) vollständig innerhalb des Materials des relevanten zweiten Abschnitts der Vorrichtung, in dem es/sie ausgebildet ist/sind, enthalten ist/sind;
oder (b) der begrenzende Umfang (die begrenzenden Umfänge) von beliebigen zwei der Löcher/Öffnungen oder Schlitze/Spalten (149a, 149b) nur teilweise innerhalb des Materials des relevanten zweiten Abschnitts der Vorrichtung (140), in dem sie ausgebildet sind, enthalten ist/sind, sodass das relevante Loch (die relevanten Löcher)/die Öffnung(en) oder Schlitze/Spalten (149a, 149b) an mindestens einer Seite oder einem Abschnitt davon offen und unbegrenzt ist/sind, wodurch eine oder eine jeweilige Lasche oder Zunge (151'a, 151'b) zwischen mindestens einem Paar davon erzeugt wird.

13. Verankerungsanordnung zum Verankern eines oder mehrerer sublaminärer Bänder oder Streifen (60a, 60b) in Bezug auf einen oder mehrere Wirbel der Wirbelsäule bei der korrigierenden Wirbelsäulenchirurgie, die Anordnung umfassend:
eine Pedikelschraube (30; 130) zum Einführen in einen ausgewählten Wirbelpedikel; und
eine Verankerungsvorrichtung (40; 140) nach einem der Ansprüche 1 bis 12;
wobei optional der Kopfabschnitt (32; 132) der Pedikelschraube (30; 130) zweite Eingriffsmittel (32Na, 32Nb; 132Fa, 132Fb) umfasst, die für einen Eingriff mit den ersten Eingriffsmitteln (42Na, 42Nb; 142a, 142b) konfiguriert sind, die an dem ersten Abschnitt (41; 141) der Verankerungsvorrichtung (40, 140) bereitgestellt sind, wodurch die ersten und zweiten Eingriffsmittel (42Na, 42Nb; 142a, 142b; 32Na, 32Nb; 132Fa, 132Fb) jeweils so konfiguriert sind, dass sie gemeinsam, sobald sie miteinander in Eingriff stehen, die Eingriffsbefestigung des ersten Abschnitts (41; 141) der Verankerungsvorrichtung (40; 140) an dem Kopfabschnitt (32; 132) der Pedikelschraube (30; 130) bewirken.

14. Kit oder Satz von Bauteilen zur Verwendung in der korrigierenden Wirbelsäulenchirurgie, das Kit oder der Satz umfassend:
mindestens eine Pedikelschraube (30; 130) zum Einführen in einen ausgewählten Wirbelpedikel eines oder mehrerer Wirbel der Wirbelsäule; und
mindestens eine Verankerungsvorrichtung (40; 140) nach einem der Ansprüche 1 bis 12;
optional zusammen mit einem oder mehreren sublaminären Bändern oder Streifen (60a, 60b) zur Verankerung in Bezug auf den einen oder die mehreren Wirbel der Wirbelsäule bei der korrigierenden Wirbelsäulenchirurgie.

## Revendications

1. Dispositif d'ancrage (40) permettant l'ancrage d'une ou plusieurs bandes ou rubans sous-laminaires (60a, 60b) à une vis pédiculaire (30), le dispositif (40) comprenant:
une première partie (41) conçue pour être fixée à une partie de tête (32) de la vis pédiculaire (30) par mise en prise avec ladite partie de tête (32); et
une pluralité de secondes parties (46a, 46b) permettant l'ancrage à celles-ci d'une ou plusieurs bandes ou rubans sous-laminaires (60a, 60b);
dans lequel la première partie (41) comporte des premiers moyens de mise en prise (42Na, 42Nb) pour une mise en prise avec la partie de tête (32) de la vis pédiculaire (30) pour effectuer ladite fixation par mise en prise de la première partie (41) du dispositif d'ancrage à celle-ci;
dans lequel ladite pluralité de secondes parties (46a, 46b) sont agencées de manière équiangulaire ou symétrique autour de la première partie (41) du dispositif (40), chaque seconde partie (46a, 46b) étant conçue et agencée pour permettre l'ancrage à celle-ci d'une dite bande ou ruban sous-laminaire (60a, 60b), et chacune de ladite seconde partie (46a, 46b) comportant, ou étant conçue comme telle, une partie ou une extension en forme d'anneau ou de boucle perforée ou creuse, située sur des côtés diamétralement opposés de la première partie (41) du dispositif ou s'étendant à partir de ceux-ci, et s'étendant dans des directions radialement opposées à partir de celle-ci,
et en outre dans lequel chacune des secondes parties (46a, 46b) a un plan général dans lequel elle se trouve et les secondes parties (46a, 46b) sont conçues de telle sorte que les plans généraux respectifs des secondes parties (46a, 46b) soient sensiblement coplanaires les uns avec les autres.

2. Dispositif d'ancrage (40) selon la revendication 1, dans lequel la première partie (41) est conçue pour être fixée à la partie de tête (32) de la vis pédiculaire (30) par mise en prise des premiers moyens de mise en prise (42Na, 42Nb) avec au moins une partie de paroi latérale radialement externe de la partie de tête de vis (32);
éventuellement dans lequel la première partie (41) est conçue de telle sorte que, lors de la mise en prise des premiers moyens de mise en prise (42Na, 42Nb) avec la partie de paroi latérale externe de la partie de tête de vis (32), au moins une partie inférieure de la première partie (41) du dispositif (40) adjacente aux premiers moyens de mise en prise (42Na, 42Nb) vienne en butée contre la partie de paroi latérale inférieure de la partie de tête de vis (32), ou soit adjacente à celle-ci, ou se mette en prise avec celle-ci.

3. Dispositif d'ancrage (40) selon la revendication 1 ou la revendication 2, dans lequel la première partie (41) du dispositif est conçue de telle sorte qu'elle puisse être fixée à la partie de tête (32) de la vis pédiculaire (30), par la mise en prise avec celle-ci des premiers moyens de mise en prise (42Na, 42Nb), au moyen de l'insertion d'une tige filetée (34) de la vis pédiculaire (30), laquelle tige (34) s'étend à partir d'une base (32L) de la partie de tête (32), à travers une ouverture (48) dans la première partie (41) du dispositif dans une direction s'éloignant de la première partie (41) du dispositif, et d'un mouvement relatif l'une vers l'autre de la première partie (41) du dispositif et de la base (32L) de la partie de tête de vis (32).

4. Dispositif d'ancrage (40) selon l'une quelconque revendication précédente, dans lequel la première partie (41) comprend une partie de base perforée ou creuse et une ou plusieurs, éventuellement au moins une paire de parties de fixation (42a, 42b) agencées de manière opposée l'une par rapport à l'autre, s'étendant de manière globalement perpendiculaire à partir de celle-ci;
éventuellement dans lequel l'une ou les deux des conditions suivantes (i) et (ii) est/sont remplie(s):
(i) dans lequel les parties de fixation (42a, 42b) sont conçues comme une paire de pattes, languettes ou bras en saillie agencés de manière diamétralement opposée (42a, 42b) s'étendant perpendiculairement à partir de la partie de base sur des côtés diamétralement opposés de celle-ci, et sont disposées de manière à être globalement sensiblement parallèles l'une à l'autre et conçues pour se mettre en prise avec la paroi latérale externe de la partie de tête (32) de la vis (30) sur des côtés diamétralement opposés de la partie de tête de vis (32);
(ii) dans lequel les parties de fixation (42a, 42b) se terminent chacune au niveau d'une extrémité distale de celles-ci (c'est-à-dire l'extrémité de celles-ci opposée à une extrémité proximale de celles-ci au niveau de laquelle la partie de fixation respective (42a, 42b) est reliée à la partie de base de la première partie (41) du dispositif) par une partie embout de mise en prise faisant saillie radialement vers l'intérieur (42Na, 42Nb), moyennant quoi les parties embout de mise en prise (42Na, 42Nb) de la paire de parties de fixation (42a, 42b) pointent généralement l'une vers l'autre à partir de côtés opposés, en particulier de côtés diamétralement opposés, de la première partie (41) du dispositif.

5. Dispositif d'ancrage (40) selon la revendication 4, dans lequel la condition (ii) est remplie, et dans lequel chaque partie embout de mise en prise (42Na, 42Nb) comporte une surface de mise en prise (44Fa, 44Fb) inclinée obliquement en dessous de celle-ci, dans lequel soit:
(a) l'angle d'inclinaison de chaque surface de mise en prise respective (44Fa, 44Fb) est tel qu'une extrémité radialement interne de chaque partie embout respective (42Na, 42Nb) est plus proche de la partie de base de la première partie (41) du dispositif qu'une extrémité radialement externe de la partie embout respective (42Na, 42Nb); soit
(b) chaque partie embout de mise en prise (42Na, 42Nb) comprend une dite surface de mise en prise (44Fa, 44Fb), qui est cette surface dirigée vers la partie de base de la première partie (41) du dispositif, qui forme un angle inférieur à 90 degrés, éventuellement un angle dans la plage allant de 50 ou 60 ou 70 à 80 ou 85 ou 87 degrés, avec une surface latérale radialement interne de la partie de fixation respective (42a, 42b) à laquelle la partie embout respective (42Na, 42Nb) est reliée.

6. Dispositif d'ancrage (40) selon soit la revendication 4, dans lequel la condition (ii) est remplie, soit la revendication 5, et dans lequel les parties embouts de mise en prise respectives (42Na, 42Nb) de la paire de parties de fixation (42a, 42b) forment un mécanisme de fixation par encliquetage avec des seconds moyens de mise en prise (32Na, 32Nb) sur la partie de tête (32) de la vis pédiculaire (30).

7. Dispositif d'ancrage (40) selon l'une quelconque revendication précédente, en outre dans lequel les secondes parties (46a, 46b) sont conçues de telle sorte que les plans généraux respectifs des secondes parties (46a, 46b) soient sensiblement coplanaires avec un plan général d'une partie de base du dispositif d'ancrage (40) à partir de laquelle s'étendent les premiers moyens de mise en prise (42Na, 42Nb) qui fournissent la fixation par mise en prise de la première partie (41) du dispositif à la partie de tête de vis pédiculaire (32).

8. Dispositif d'ancrage (40) selon l'une quelconque revendication précédente, dans lequel chacune des secondes parties (46a, 46b) a un insert (50a, 50b) monté dans celle-ci afin de fournir le moyen d'ancrage à cette seconde partie (46a, 46b) du dispositif de la ou des bandes ou rubans sous-laminaires respectifs (60a, 60b);
et dans lequel le ou chaque insert (50a, 50b) comprend un corps central (51a, 51b), dans lequel est prévue une fente ou une ouverture (49a, 49b) à travers laquelle peut passer ou être logée la ou les bandes ou rubans sous-laminaires respectifs (60a, 60b), et un cadre externe conçu pour permettre le montage de l'insert (50a, 50b) dans la seconde partie respective (46a, 46b) du dispositif,
éventuellement dans lequel le ou chaque cadre externe est conçu pour pouvoir se mettre en prise avec la seconde partie respective (46a, 46b) du dispositif au moyen d'une liaison par encliquetage respective.

9. Dispositif d'ancrage (40) selon la revendication 8, dans lequel l'une ou les deux des conditions suivantes (i) et (ii) est/sont remplie(s):
(i) dans lequel le corps central (51a, 51b) du ou des inserts respectifs (50a, 50b) se présente sous la forme d'un cylindre circulaire court, qui est monté de manière à pouvoir tourner librement à l'intérieur d'un montage circulaire de géométrie similaire à l'intérieur du cadre respectif, de sorte que l'orientation de la fente ou de l'ouverture (49a, 49b) peut varier dans celui-ci ou s'ajuster automatiquement de manière à adopter une orientation optimale telle que dictée par les différentes forces qui peuvent être exercées sur le dispositif d'ancrage (40) par la ou les bandes ou rubans sous-laminaires (60a, 60b) une fois qu'elles ont été ancrées au dispositif et serrées dans leur position sécurisée ou verrouillée souhaitée;
(ii) dans lequel soit:
(a) la fente ou l'ouverture (49a, 49b) à l'intérieur du corps central respectif (51a, 51b) de chaque insert respectif (50a, 50b) est dimensionnée pour permettre à la ou les bandes ou rubans sous-laminaires respectifs (60a, 60b) de passer et de coulisser librement à travers celle-ci, et un dispositif de fixation ou de verrouillage distinct séparé est prévu pour maintenir la ou les bandes ou rubans sous-laminaires respectifs (60a, 60b) dans leur position serrée une fois qu'ils ont été déployés dans leur configuration souhaitée, après avoir été passés ou enfilés autour de la ou des vertèbres concernées et ancrés au dispositif d'ancrage (40); soit
(b) la ou chaque fente ou ouverture respective (49a, 49b) à l'intérieur du corps central respectif (51a, 51b) de l'insert respectif (50a, 50b) comporte elle-même un dispositif de fixation ou de verrouillage qui permet à la bande ou au ruban sous-laminaire respectif (60a, 60b) d'être fixé directement à l'intérieur de l'insert respectif (50a, 50b) du dispositif.

10. Dispositif d'ancrage (140) selon la revendication 1 ou la revendication 2, ou selon la revendication 4 ou la revendication 7 lorsqu'elles dépendent de la revendication 1 ou de la revendication 2, dans lequel la première partie du dispositif (140) est conçue de telle sorte qu'elle puisse être fixée à la partie de tête (132) de la vis pédiculaire (130), par le biais de la mise en prise avec celle-ci des premiers moyens de mise en prise (142a, 142b), au moyen du placement d'un trou ou d'une ouverture (148) dans la première partie du dispositif (140) au-dessus de la partie de tête (132) de la vis pédiculaire depuis le dessus de celle-ci, c'est-à-dire depuis le côté de celle-ci opposé à celui d'où s'étend la tige filetée (134) de la vis pédiculaire, et le dispositif d'ancrage (140) et la partie de tête (132) de la vis pédiculaire (130) peuvent être fixés et assemblés grâce à une liaison par emboîtement, par friction ou par butée.

11. Dispositif d'ancrage (140) selon la revendication 10, dans lequel l'une quelconque ou plusieurs des conditions suivantes (i), (ii) et (iii) est/sont remplie(s):
(i) dans lequel le dispositif d'ancrage (140) est réalisé sous la forme d'une structure monobloc sensiblement unitaire,
éventuellement dans lequel le dispositif d'ancrage (140) est conçu avec au moins une partie (142a) de celui-ci courbée ou pliée ou concave ou convexe ou en pente ou en gradins par rapport au reste du dispositif (140);
(ii) dans lequel les premiers moyens de mise en prise (142a, 142b) comprennent au moins une paire de parties à bride ou d'appui ou de roulement (142a, 142b) du dispositif, agencées de manière opposée ou diamétralement opposée;
(iii) dans lequel chaque seconde partie du dispositif (140) comprend un ou plusieurs, ou une pluralité de, trous ou ouvertures ou fentes ou entailles (146a, 146b; 149a, 149b) formés dans et/ou s'étendant à travers le matériau de la seconde partie respective.

12. Dispositif d'ancrage (140) selon la revendication 11, dans lequel la condition (iii) est remplie, et dans lequel:
soit (a) le ou les périmètres de délimitation de l'un quelconque ou de plusieurs desdits trous/ouvertures ou fentes/entailles (146a, 146b) sont entièrement contenus à l'intérieur du matériau de la seconde partie concernée du dispositif dans laquelle ils sont formés;
soit (b) le ou les périmètres de délimitation de deux quelconques desdits trous/ouvertures ou fentes/entailles (149a, 149b) ne sont que partiellement contenus à l'intérieur du matériau de la seconde partie concernée du dispositif (140) dans laquelle ils sont formés, de sorte que le ou les trous/ouvertures ou fentes/entailles (149a, 149b) est/sont ouvert(s) et sans limite sur au moins un côté ou une partie de celui-ci, créant ainsi une languette ou une patte respective (151'a, 151'b) entre au moins une paire de ceux-ci.

13. Agencement d'ancrage permettant l'ancrage d'une ou plusieurs bandes ou rubans sous-laminaires (60a, 60b) par rapport à une ou plusieurs vertèbres dans le cadre d'une chirurgie correctrice de la colonne vertébrale, l'agencement comprenant:
une vis pédiculaire (30; 130) destinée à être insérée dans un pédicule vertébral sélectionné; et
un dispositif d'ancrage (40; 140) selon l'une quelconque des revendications 1 à 12;
éventuellement dans lequel la partie de tête (32; 132) de la vis pédiculaire (30; 130) comprend des seconds moyens de mise en prise (32Na, 32Nb; 132Fa, 132Fb) conçus pour une mise en prise avec les premiers moyens de mise en prise (42Na, 42Nb; 142a, 142b) prévus sur la première partie (41; 141) du dispositif d'ancrage (40, 140), moyennant quoi les premiers et seconds moyens de mise en prise (42Na, 42Nb; 142a, 142b; 32Na, 32Nb; 132Fa, 132Fb) sont chacun conçus de sorte qu'ensemble, une fois qu'ils sont mis en prise les uns avec les autres, ils effectuent ladite fixation par mise en prise de la première partie (41; 141) du dispositif d'ancrage (40; 140) à la partie de tête (32; 132) de la vis pédiculaire (30; 130).

14. Kit ou ensemble de parties composantes destinées à être utilisées dans le cadre d'une chirurgie correctrice de la colonne vertébrale, le kit ou ensemble comprenant:
au moins une vis pédiculaire (30; 130) destinée à être insérée dans un pédicule vertébral sélectionné d'une ou plusieurs vertèbres; et
au moins un dispositif d'ancrage (40; 140) selon l'une quelconque des revendications 1 à 12;
éventuellement conjointement avec une ou plusieurs bandes ou rubans sous-laminaires (60a, 60b) permettant l'ancrage par rapport à ladite une ou auxdites plusieurs vertèbres dans le cadre de ladite chirurgie correctrice de la colonne vertébrale.
